# EUROPEAN PATENT APPLICATION

(11) **EP 2 420 146 A1**
(43) Date of publication of application: **22.02.2012**
(21) Application number: 11174389.4
(22) Date of filing: 29.08.2007
(51) Int. Cl.: A23L 1/29, A23L 1/30, A61K 31/202, A61K 31/66

(54) **Use of DPA(n-6) oils in infant formula**

(30) Priority: 29.08.2006 US 823875 P
(62) Divisional of application: 07814550.5
(71) Applicant: MARTEK BIOSCIENCES CORPORATION, Columbia, MD 21045 (US)
(72) Inventor: Arterburn, Linda, Ellicott City Maryland 21043-6614 (US); Barclay, William, Boulder Colorado 80303 (US); Flatt, James, Colorado Springs Colorado 80906 (US); van Elswyk, Mary, Longmont Colorado 80503 (US); Zeller, Samuel, G., Lexington Massachusetts 02421 (US)
(74) Representative: O'Brien, Simon Warwick

(57) **Abstract**

Infant formula compositions containing docosapentaenoic acid n-6 ("DPA(n-6)") and other polyunsaturated fatty acids and methods for their preparation and use are provided.

## Description

### FIELD OF THE INVENTION

The invention relates to a method of preparing infant formulas with polyunsaturated fatty acids and the corresponding infant formula compositions.

### BACKGROUND OF THE INVENTION

It is desirable to increase the dietary intake of the beneficial long chain polyunsaturated fatty acids (LC-PUFAs), including for example, omega-3 long chain polyunsaturated fatty acids (omega-3 LC-PUFAs), and omega-6 long chain polyunsaturated fatty acids (omega-6 LC-PUFA) for infants and toddlers. As used herein, reference to a long chain polyunsaturated fatty acid or LC-PUFA, refers to a polyunsaturated fatty acid having 18 or more carbons. Recognition of clinical benefits attributed to omega-6 and omega-3 long-chain polyunsaturated fatty acids (LC-PUFA) has stimulated efforts to increase the use of these fatty acids in infant diets (Carlson and Forsythe 2001 Curr. Op. Clin Nutr. Metab. Care 4, 123-126; Birch et al. 2000 Develop. Med. Child Neuro. 42, 174-181). Furthermore, the clinical benefits of omega-3 LC-PUFA for maternal supplements, and other types of nutritional supplements and foods are also well recognized (Barclay and Van Elswyk 2000, FUNCTIONAL FOODS 2000, Angus, F. and Miller C., eds., pp. 60-67, Leatherhead Publishing, Surrey).

Fatty acids are classified based on the length and saturation characteristics of the carbon chain. As used herein, fatty acids include fatty acids in various forms, including but not limited to triacylglycerols, diacylglycerols, monoacylglycerols, phospholipids, free fatty acids, esterified-fatty acids, and natural or synthetic derivative forms of these fatty acids (e.g. calcium salts of fatty acids, ethyl esters, etc). Short chain fatty acids have 2 to about 7 carbons and are typically saturated. Medium chain fatty acids have from about 8 to about 17 carbons and may be saturated or unsaturated. Long chain fatty acids have from 18 to 24 or more carbons and may also be saturated or unsaturated. In longer chained fatty acids there may be one or more points of unsaturation, giving rise to the terms "monounsaturated" and "polyunsaturated," respectively. Long chain PUFAs (LC-PUFAs) are of particular interest in the present invention.

LC-PUFAs are categorized according to the number and position of double bonds in the fatty acids according to a well understood nomenclature. There are two common series or families of LC-PUFAs, depending on the position of the double bond closest to the methyl end of the fatty acid: the n-3 (or m-3 or omega-3) series contains a double bond at the third carbon, while the n-6 (or ω-6 or omega-6) series has no double bond until the sixth carbon. Thus, docosahexaenoic acid ("DHA") has a chain length of 22 carbons with 6 double bonds beginning with the third carbon from the methyl end and is designated "22:6 n-3". Other important omega-3 LC-PUFAs include eicosapentaenoic acid ("EPA") which is designated "20:5 n-3" and docosapentaenoic acid n-3 ("DPA(n-3)") which is designated "22:5 n-3." In addition, omega-6 LC-PUFAs are used in connection with the present invention. For example, arachidonic acid ("ARA") which is designated "20:4 n-6" and docosapentaenoic acid n-6 ("DPAn-6") which is designated "22:5 n-6" are suitable.

*De novo* or "new" synthesis of omega-3 fatty acids and omega-6 fatty acids does not occur in the human body. The precursor fatty acid for the omega-3 and omega-6 fatty acids are alpha-linolenic acid (18:3n-3) and linoleic acid (18:2n-6), respectively. These fatty acids are essential fatty acids and must be consumed in the diet because humans cannot synthesize them. Humans cannot insert double bonds closer to the omega end than the seventh carbon atom counting from that end of the molecule. However, the body can convert alpha-linolenic acid and linoleic acid to LC PUFAs such as DHA and ARA, respectively, although at very low efficiency. All metabolic conversions occur without altering the omega end of the molecule that contains the omega-3 and omega-6 double bonds. Consequently, omega-3 and omega-6 acids are two separate families of fatty acids since they are not interconvertible in the human body.

Both term and preterm infants can synthesize the LC-PUFAs from the respective essential fatty acids, but controversy has centered around the fact that breastfed infants have higher plasma concentrations of these LC-PUFAs than formula-fed infants. This information could be interpreted to imply that formula-fed infants cannot synthesize enough of these fatty acids to meet ongoing needs, though the plasma content of DHA and ARA is only a very small fraction of the total fatty acid pool available in other tissues. It has been demonstrated that the addition of DHA to infant formula improves infant visual function, and that the addition of both DHA and ARA improves cognitive development and facilitates normal infant growth. Sources of oils containing both DHA and DPA(n-6), and oil containing ARA, have been developed for nutritional use and these have been suggested for use in infant formula to better match the LC-PUFA profile found in human breast milk.

Infant formulas that use tuna oil or egg lipids as a source of DHA and ARA may contain very small amounts of DPA(n-6). Use of egg yolk oils and fish oils in infant formula was proposed by Clandinin U.S. Pat. No. 4,670,285. He reported (Table 2) that human breast milk provides about 1.7 mg DPA(n-6) per 100 mls representing about 0.07% of total fatty acids in human milk.

Arachidonic acid, along with its elongation products docosatetraenoic acid and docosapentaenoic acid, has been suggested for inclusion in infant diets along with docosahexaenoic acid in recognition of their natural occurrence in human breast milk (Specter 1994). DPA(n-6) is found in human breast milk at approximately 0.1 % of total fatty acids (Koletzko et al. 1992, J. Pediatrics. 120, S62-S70). DPA(n-6) is typically a component of tissues in the human body, including the heart (Rocquelin et al. 1989 Lipids 24, 775-780), brain (Svennerholm et al. 1978, J. Neurochem. 30, 1383-1390; O'Brien et al. 1965 J. Lipid Res. 6, 545-551), liver (Salem 1989 Omega-3 Fatty Acids: Molecular and Biochemical Aspects, in NEW PROTECTIVE ROLES FOR SELECTED NUTRIENTS, (Spiller, G. A. and Scala, J., eds.), pp.109-228, Alan R Liss, New York.), red blood cells (Sanders et al. 1978 Am. J. Clin. Nutr. 31, 805-813; Sanders et al. 1979 Br. J. Nutr. 41, 619-623) and adipose tissue (Clandinin et al. 1981, Early Human Development 5, 355-366). DPA(n-6) represents 9% of the long chain omega-6 fatty acids in the cortex of the human brain, and 5% of long chain omega-6 fatty acids in the retina of the eye (Makrides et al. 1994 Am. J. Clin. Nutr. 60, 189-194). In humans, DHA and DPA(n-6) represent the final elongation products in the n-3 and n-6 fatty acid pathways, respectively. Many of the same dietary sources of DHA for humans also contain DPA (n-6). Major sources of DPA(n-6) in the diet for adults and children are poultry (meat and eggs) and seafood (Taber et al. 1998 Lipids 33(12), 1151-1157; Nichols et al. 1998 SEAFOOD: THE GOOD FOOD: THE OIL (FAT) CONTENT AND COMPOSITION OF AUSTRALIAN COMMERCIAL FISHES, SHELLFISHES AND CRUSTACEANS. CSIRO Marine Research, Hobart, Australia).

A number of organizations provide recommendations for levels of LC-PUFAs in infant formulas. For example, the International Society for the Study of Fatty Acids and Lipids (ISSFAL) recommended in 1994 that infant formulas provide 60-100 mglltg/day as preformed arachidonic acid and its associated long chain omega-6 forms (22:4(n-6) and 22:5(n-6)). ISSFAL Board of Directors, ISSFAL Newsletter :4-5 (1994). ISSFAL made the following recommendations for LC-PUFAs in infant formula in 1999 in order to insure adequate intake of the LC-PUFAs: linoleic acid, 18:2n-6, 10%; α-linolenic acid, 18:3, 1.50%; arachidonic acid, 20:4n-6, 0.50%; docosahexaenoic acid, 22:6n-3, 0.35%; eicosapentaenoic acid, 20:5n-3, 0.10%. There was no recommendation for DPA(n-6) in this set of recommendations. (*See,* Simopoulos et al., J Am Coll Nutr 18 (5): 487 (1999)).

Established Recommended Daily Intakes (RDIs) for the long chain omega-3 fatty acids (DHA and EPA) range from 200 mg/day (COMA (Committee on Medical Aspects of Food Policy) (1994). Annual Report. London: Department of Health) to 1.2 g/day (Nordic Council of Ministers (1989). Nordic Nutrition Recommendations, Second Edition). These RDIs represent a range of DHA/EPA intakes from 3 to 20 mg DHA+EPA/kg/day for adults. While an RDI for DPA(n-6) has not been established, a reference intake for comparison purposes can be gleaned from the data on the DPA(n-6) content in human breast milk. Based on a reported average level of approximately 0.1% (of total fatty acids) of DPA(n-6) in breast milk (Carlson et al. 1986 Am.J. Clin. Nutr. 44, 798-804), a 3 kg breast feeding infant consuming 0.8-1.0 liter of millc/day that contains 32 g/liter of fat would consume approximately 26-32 mg/day of DPA(n-6), or approximately 10 mg DPA (n-6)/kg/day. Related to combined DHA and DPA(n-6) intake in foods, it is well known that the PUFA content of human breast milk is an indicator of the fatty acid composition of the maternal diet (Chen et al. 1994 Lipids 30, 15-21; Jensen 1996 Prog. Lipid Res. 35, 53-91). Chen et al. Lipids 32, 1061-1067 (1997) recently examined the differences in breast milk fatty acid composition between a population of Chinese women on a "westernized" diet versus a population on a "traditional" Chinese diet. Women on the "traditional" diet consumed a daily average of 7 eggs, 220 g chicken and 54 g of pork and 11 g of fish. This would represent a daily DPA(n-6) intake of about 158 mg/day based on the data of Taber (1998) and Nichols et al. (1998). Women on the "westernized" diet consumed daily 1 egg, 44 g chicken, 29 g pork, and 26 g fish representing a DPA(n-6) intake of about 32 mg/day (Taber 1998; Nichols et al. 1998). Although DPA(n-6) in the traditional diet was estimated to be five times higher than in the westernized diet, the DPA(n-6) content (0.10 wt% total fatty acids) of the breast milk of the women on the "traditional" diet was not significantly different than that of the women on the "westernized" diet (0.09 wt% total fatty acids). ARA levels were higher in the breast milk of women on the traditional diet (0.76 vs 0.61 wt% total fatty acids) but were not statistically different. This indicates that there is minimal accumulation of DPA(n-6) when consumed in combination with DHA and that DPA(n-6) may help maintain ARA levels. Most importantly in infants, this high level of intake of DPA(n-6) (in terms of mg/kg body weight) occurs during the period when brain and neural tissues are in rapid development. This is also the time when DHA accumulation peaks in the infant with the DHA content of human breast milk several times higher than that of DPA(n-6).

DPA(n-6) is known to be able to retroconvert to arachidonic acid via p-oxidation (Stoffel et al. 1970 Hoppe-Seyler's Z. Physiol. Chem. 351, 1545-1554). Retroconversion of DPA(n-6) to ARA is not a reverse chain elongation, but rather a partial degradation including a hydrogenation and chain shortening, as determined by experiments with isotope-labeled fatty acids in rat liver preparations. It has been suggested that feeding DPA(n-6) concomitant with DHA avoids a rapid decline of plasma ARA that is seen when DHA alone is administered. It has not been suggested that ARA feeding could be decreased, however.

ARA is generally the LC-PUFA added in the highest concentration to infant formula. Current recommendations from health and regulatory organizations suggest that ARA and DHA should be added to infant formula in an approximate ratio of about 2:1-1:1 (ARA:DHA). It is known that high dietary intake of omega-3 LC-PUFAs such as DHA results in an increase in DHA content, but also reduces plasma levels of ARA. Thus, ARA is added to infant formulas at these levels to compensate for the decline in ARA plasma levels resulting from DHA administration. Infant formulas containing LC-PUFAs can be more expensive than standard infant formulas, due to the added cost of the LC-PUFA ingredients.

It would be desirable to enrich infant formula in a manner that provides the benefits of supplementation of DHA and ARA with increased production efficiency. These and other needs are answered by the present invention.

### SUMMARY OF THE INVENTION

The present invention provides infant formulas having ARA levels which are lower than current recommended levels or targets, but which nonetheless significantly reduce or prevent the associated decline in ARA plasma levels that will occur when the infant formula contains an omega-3 fatty acid such as DHA. In addition, the present invention provides infant formulas having ARA levels that are at about current recommended levels or targets, but which provide augmented plasma ARA levels compared to current products. ARA is important for normal growth, weight, immune development and nervous system development; thus, reducing or preventing the decline in ARA plasma levels maintains normal infant growth, weight, immune system development, and nervous system development. Infant formula that contains an omega-3 fatty acid such as DHA causes decreases in plasma levels of ARA in infants, and consequently, ARA is added to infant formula to avoid this decrease. In some embodiments, the present invention provides a method for enriching DHA supplemented infant formula with DPA(n-6), a fatty acid that is generally present in breast milk, to compensate for ARA plasma level decreases due to DHA. This is described in detail below.

In one embodiment, the invention provides an infant formula composition, wherein, when ready for consumption by the infant, the composition comprises long chain n-3 fatty acids and long chain n-6 fatty acids, and in which the long chain n-3 fatty acids comprise docosahexaenoic acid (DHA); the long chain n-6 fatty acids comprise docosapentaenoic acid (DPA(n-6)) and optionally arachidonic acid (ARA). In this embodiment, the ratio of ARA:DHA is less than about 3:1, and the DHA:DPA(n-6) ratio is from about 5:1 to about 0.5:1.

The invention also provides a method of preparing an infant formula composition, comprising combining nutritional components, long chain n-3 fatty acids and long chain n-6 fatty acids; wherein the long chain n-3 fatty acids comprise DHA; wherein the long chain n-6 fatty acids comprise ARA and DPA(n-6); wherein the ratio of ARA:DHA is less than about 3:1, and wherein the DHA:DPA(n-6) ratio is from about 5:1 to about 0.5:1. Infant formula compositions prepared by this method are also included in the invention.

In certain aspects of these embodiments, the ratio of ARA:DHA is less than about 2.95:1, less than about 2.85:1, less than about 2.9:1, less than about 2.8:1, less than about 2.75:1, less, than about 2.5:1, less than about 2.25:1, less than about 2:1, less than about 1.95:1, less than about 1.9:1, less than about 1.85:1, less than about 1.8:1, or less than about 1.75:1. In other embodiments, the ratio of ARA:DHA is greater than about 0.4:1. In still other embodiments, the composition does not comprise ARA.

In certain aspects of these embodiments, the DHA:DPA(n-6) ratio is from about 5:1 to about 3:1, from about 5:1 to about 2:1, from about 5:1 to about 1:1, about 4:1, about 3:1, about 2:1, about 1:1, or about 0.5:1.

In certain aspects of these embodiments, the long chain n-6 fatty acids in the infant formula compositions are supplied in a source oil, wherein the source oil comprises long chain n-6 fatty acids comprising at least about 2% by weight DPA(n-6). In other embodiments, the source oil comprises long chain n-6 fatty acids comprising at least about 5% by weight DPA(n-6), at least about 10% by weight DPA(n-6), at least about 15% by weight DPA(n-6), at least about 20% by weight DPA(n-6), at least about 25% by weight DPA(n-6), at least about 30% by weight DPA(n-6), at least about 40% by weight DPA(n-6), or at least about 50% by weight DPA(n-6).

In certain aspects of these embodiments, the composition comprises at least about 7 mg/L of DPA(n-6), at least about 13 mg/L of DPA(n-6), at least about 26 mg/L of DPA(n-6), at least about at least about 40 mg/L of DPA(n-6), at least about 53 mg/L of DPA(n-6), at least about 66 mg/L of DPA(n-6), at least about 80 mg/L of DPA(n-6), at least about 100 mg/L of DPA(n-6), at least about 120 mg/L of DPA(n-6). In other embodiments, the composition comprises less than about 240 mg/L of DPA(n-6), less than about 220 mg/L of DPA(n-6), or less than about 200 mg/L of DPA(n-6). In other embodiments, the composition comprises various ranges of DPA(n-6) formed by any combination of any of the foregoing minimum and maximum values.

In certain aspects of these embodiments, the infant formula composition comprises at least 20 mg/L of DHA or at least 40 mg/L DHA. In other embodiments, the infant formula composition comprises from about 40 to about 140 mg/L of DHA, from about 20 to about 200 mg/L of DHA. In other embodiments, the composition comprises less than about 140 mg/L of DHA or less than about 200 mg/L of DHA. In other embodiments, the composition comprises various ranges of DHA formed by any combination of any of the foregoing minimum and maximum values.

In certain aspects of these embodiments, the infant formula composition comprises from about 0.37 mg/L of cholesterol to about 500 mg/L cholesterol. In other embodiments, the infant formula composition comprises from about 0.37 mg/L of cholesterol to about 300 mg/L cholesterol, from about 0.37 mg/L of cholesterol to about 100 mg/L cholesterol, from about 0.37 mg/L of cholesterol to about 50 mg/L cholesterol, or from about 0.37 mg/L of cholesterol to about 10 mg/L cholesterol. In certain aspects of these embodiments, the infant formula composition comprises from about 0.37 ing/L of cholesterol to about 3.75 mg/L cholesterol. In some embodiments, the infant formula composition comprises greater than about 0.5 mg/L cholesterol. In some embodiments, the infant formula composition comprises about 0.37 mg/L cholesterol, about 0.75 mg/L cholesterol, about 1.5 mg/L cholesterol, about 2.3 mg/L cholesterol, about 3 mg/L cholesterol, or about 3.75 mg/L cholesterol. In certain aspects the cholesterol is provided in the form of a microbial oil, the oil containing a long chain omega-3 or long chain omega-6 fatty acid.

In certain aspects of these embodiments, the infant formula composition comprises eicosapentaenoic acid (EPA) in an amount less than about 3 mg/L. In certain aspects, the infant formula composition comprises EPA in an amount less than about 60 mg/L. In other aspects, the infant formula composition comprises from about 15 mg/L to about 30 mg/L EPA. In some embodiments, the EPA:DHA ratio is provided in a ratio of up to 1:1.

In certain aspects of these embodiments, the infant formula composition is formulated to provide at least about 2 mg/lkg/day DPA(n-6) when administered to an infant. In some embodiments, the infant formula composition is formulated to provide from about 3 mg/kg/day DPA(n-6) to about 8 mg/kg/day DPA(n-6) when administered to an infant.

In a further embodiment, the invention provides an infant formula composition comprising long chain n-6 fatty acids and DHA, in which the long chain n-6 fatty acids in the infant formula compositions are supplied in a source oil, wherein the source oil comprises long chain n-6 fatty acids comprising at least about 2% by weight DPA(n-6), the DHA:DPA(n-6) ratio in the infant formula compositions is from about 10:1 1 to about 0.5:1, and wherein the in the infant formula compositions are formulated to provide at least about 2 mg/kg/day DPA(n-6) when administered to an infant.

The invention also provides a method of preparing an infant formula composition, comprising combining nutritional components, DHA, and the long chain n-6 fatty acids, wherein the long chain n-6 fatty acids in the infant formula compositions are supplied in a source oil, wherein the source oil comprises long chain n-6 fatty acids comprising at least about 2% by weight DPA(n-6), wherein the DHA:DPA(n-6) ratio in the infant formula compositions is from about 5:1 to about 0.5:1. The method is designed to provide a formulation that provides at least about 2 mg/kg/day DPA(n-6) when administered to an infant. Infant formula compositions prepared by this method are also included in the invention.

In certain aspects of these embodiments, the long chain n-6 fatty acids in the infant formula compositions are supplied in a source oil, wherein the source oil comprises long chain n-6 fatty acids comprising at least about 2% by weight DPA(n-6). In other embodiments, the source oil comprises long chain n-6 fatty acids comprising at least about 5% by weight DPA(n-6), at least about 10% by weight DPA(n-6), at least about 15% by weight DPA(n-6), at least about 20% by weight DPA(n-6), at least about 25% by weight DPA(n-6), at least about 30% by weight DPA(n-6), at least about 40% by weight DPA(n-6), or at least about 50% by weight DPA(n-6).

In other aspects of these embodiments, the long chain n-6 fatty acids comprise ARA, and the ratio of ARA:DHA is less than about 3:1. In some embodiments, the ratio of ARA:DHA is less than about 2.95:1, less than about 2.85:1, less than about 2.9:1, less than about 2.8:1, less than about 2.75:1, less, than about 2.5:1, less than about 2.25:1, less than about 2:1, less than about 1.95:1, less than about 1.9:1, less than about 1.85:1, less than about 1.8:1, or less than about 1.75:1. In other embodiments, the ratio of AR.A:DHA is greater than about 0.4:1. In still other embodiments, the compos ition does not comprise ARA.

In certain aspects of these embodiments, the composition comprises at least about 7 mg/L of DPA(n-6), at least about 13 mg/L of DPA(n-6), at least about 26 mg/L of DPA(n-6), at least about at least about 40 mg/L of DPA(n-6), at least about 53 mg/L of DPA(n-6), or at least about 66 mg/L of DPA(n-6), at least about 80 mg/L of DPA(n-6), at least about 100 mg/L of DPA(n-6), at least about 120 mg/L of DPA(n-6). In other embodiments, the composition comprises less than about 240 mg/L of DPA(n-6), less than about 220 mg/L of DPA(n-6), or less than about 200 mg/L of DPA(n-6).

In certain aspects of these embodiments, the infant formula composition comprises at least 20 mg/L of DHA or at least 40 mg/L DHA. In other embodiments, the infant formula composition comprises from about 40 to about 140 mg/L of DHA, from about 20 to about 200 mg/L of DHA. In other embodiments, the composition comprises less than about 140 mg/L of DHA or less than about 200 mg/L of DHA.

In certain aspects of these embodiments, the DHA:DPA(n-6) ratio in the infant formula composition is from about 5:1 to about 3:1, from about 5:1 to about 2:1, from about 5:1 to about 1:1, about 4:1, about 3:1, about 2:1, about 1:1, or about 0.5:1.

In certain aspects of these embodiments, the infant formula composition comprises from about 0.37 mg/L of cholesterol to about 500 mg/L cholesterol. In other embodiments, the infant formula composition comprises from about 0.37 mg/L of cholesterol to about 300 mg/L cholesterol, from about 0.37 mg/L of cholesterol to about 100 mg/L cholesterol, from about 0.37 mg/L of cholesterol to about 50 mg/L cholesterol, or from about 0.37 mg/L of cholesterol to about 10 mg/L cholesterol. In other aspects of these embodiments, the infant formula composition comprises from about 0.37 mg/L of cholesterol to about 3.75 mg/L cholesterol. In some embodiments, the infant formula composition comprises greater than about 0.5 mg/L cholesterol. In some embodiments, the infant formula composition comprises about 0.37 mg/L cholesterol, about 0.75 mg/L cholesterol, about 1.5 mg/L cholesterol, about 2.3 mg/L cholesterol, about 3 mg/L cholesterol, or about 3.75 mg/L cholesterol. In certain aspects the cholesterol is provided in the form of a microbial oil, the oil containing a long chain omega-3 or omega-6 long chain fatty acid.

In certain aspects of these embodiments, the infant formula composition comprises eicosapentaenoic acid (EPA) in an amount less than about 3 mg/L. In other aspects, the infant formula composition comprises EPA in an amount less than about 60 mg/L. In other aspects, the infant formula composition comprises from about 15 mg/L to about 30 mg/L EPA. In some embodiments, the EPA:DHA ratio is provided in a ratio of up to about 1:1.

In certain aspects of these embodiments, the infant formula composition is formulated to provide from about 3 mg/kg/day DPA(n-6) to about 8 mg/kg/day DPA(n-6) when administered to an infant.

In a further embodiment, the invention provides an infant formula composition formulated to comprise at least about 7 mg/L of DPA(n-6) when ready for consumption, wherein the composition further comprises long chain n-3 fatty acids; wherein the long chain n-3 fatty acids comprise DHA; wherein the composition further optionally comprises arachidonic acid ARA; and wherein the ratio of ARA:DHA is less than about 3:1.

The invention also provides a method of preparing an infant formula composition, comprising combining nutritional components, DPA(n-6), DHA, and optionally, ARA; wherein the composition is formulated to comprise at least about 7 mg/L of DPA(n-6) when ready for consumption, and wherein the ratio of ARA:DHA is less than about 3:1. Infant formula compositions prepared by this method are also included in the invention.

In certain aspects of these embodiments, the ratio of ARA:DHA is less than about 3:1. In some embodiments, the ratio of ARA:DHA is less than about 2.95:1, less than about 2.85:1, less than about 2.9:1, less than about 2.8:1, less than about 2.75:1, less, than about 2.5:1, less than about 2.25:1, less than about 2:1, less than about 1.95:1, less than about 1.9:1, less than about 1.85:1, less than about 1.8:1, or less than about 1.75:1. In other embodiments, the ratio of ARA:DHA is greater than about 0.4:1. In still other embodiments, the composition does not comprise ARA.

In certain aspects of these embodiments, the infant formula composition comprises at least about 7 mg/L of DPA(n-6), at least about 13 mg/L of DPA(n-6), at least about 26 mg/L of DPA(n-6), at least about at least about 40 mg/L of DPA(n-6), at least about 53 mg/L of DPA(n-6), or at least about 66 mg/L of DPA(n-6) at least about 80 mg/L of DPA(n-6), at least about 100 mg/L of DPA(n-6), at least about 120 mg/L of DPA(n-6). In other embodiments, the infant formula composition comprises less than about 240 mg/L of DPA(n-6), less than about 220 mg/L of DPA(n-6), or less than about 200 mg/L of DPA(n-6).

In certain aspects of these embodiments, the long chain n-6 fatty acids in the infant formula compositions are supplied in a source oil, wherein the source oil comprises long chain n-6 fatty acids comprising at least about 2% by weight DPA(n-6). In other embodiments, the long chain n-6 fatty acids in the infant formula compositions are supplied in a source oil, wherein the source oil comprises long chain n-6 fatty acids comprising at least about 5% by weight DPA(n-6), at least about 10% by weight DPA(n-6), at least about 15% by weight DPA(n-6), at least about 20% by weight DPA(n-6), at least about 25% by weight DPA(n-6), at least about 30% by weight DPA(n-6), at least about 40% by weight DPA(n-6), or at least about 50% by weight DPA(n-6).

In certain aspects of these embodiments, the infant formula composition comprises at least 20 mg/L of DHA or at least 40 mg/L DHA. In other embodiments, the infant formula composition comprises from about 40 to about 140 mg/L of DHA, from about 20 to about 200 mg/L of DHA. In other embodiments, the composition comprises less than about 140 mg/L of DHA or less than about 200 mg/L of DHA.

In certain aspects of these embodiments, the DHA:DPA(n-6) ratio in the infant formula composition is from about 10:1 to about 3:1, from about 5:1 to about 2:1, from about 5:1 to about 1:1, about 4:1, about 3:1, about 2:1, about 1:1, or about 0.5:1.

In certain aspects of these embodiments, the infant formula composition comprises from about 0.37 mg/L of cholesterol to about 500 mg/L cholesterol. In other embodiments, the infant formula composition comprises from about 0.37 mg/L of cholesterol to about 300 mg/L cholesterol, from about 0.37 mg/L of cholesterol to about 100 mg/L cholesterol, from about 0.37 mg/L of cholesterol to about 50 mg/L cholesterol, or from about 0.37 mg/L of cholesterol to about 10 mg/L cholesterol. In certain aspects of these embodiments, the infant formula composition comprises from about 0.37 mg/L of cholesterol to about 3.75 mg/L cholesterol. In some embodiments, the infant formula composition comprises greater than about 0.5 mg/L cholesterol. In some embodiments, the infant formula composition comprises about 0.37 mg/L cholesterol, about 0.75 mg/L cholesterol, about 1.5 mg/L cholesterol, about 2.3 mg/L cholesterol, about 3 mg/L cholesterol, or about 3.75 mg/L cholesterol. In certain aspects the cholesterol is provided in the form of a microbial oil, the oil containing a long chain omega-3 or omega-6 long chain fatty acid.

In certain aspects of these embodiments, the infant formula composition comprises eicosapentaenoic acid (EPA) in an amount less than about 3 mg/L. In other aspects, the infant formula composition comprises EPA in an amount less than about 60 mg/L. In other aspects, the infant formula composition comprises from about 15 mg/L to about 30 mg/L EPA. In some embodiments, the EPA:DHA ratio is provided in a ratio of up to 1:1.

In other aspects of these embodiments, the infant formula composition is formulated to provide at least about 2 mg/kg/day DPA(n-6) when administered to an infant. In some embodiments, the infant formula composition is formulated to provide from about 3 mg/kg/day DPA(n-6) to about 8 mg/kg/day DPA(n-6) when administered to an infant.

In yet another embodiment the invention provides an infant formula composition and a method for preparing an infant formula composition in which the amount of DPA(n-6) in the composition is determined based on the ARA plasma levels that are desired. In this embodiment, the infant formula composition is formulated to meet DHA and ARA target requirements, and the composition comprises DHA in the target DHA requirement amount, ARA in a sub-target amount less than the target ARA requirement amount; and DPA(n-6) in an amount sufficient to retroconvert to ARA *in vivo,* in an amount sufficient to offset the ARA sub-target amount.

In another embodiment, the invention provides an infant formula composition and a method for preparing an infant formula composition formulated to meet DHA and ARA target requirements, wherein the composition comprises DHA in the target DHA requirement amount; ARA in a sub-target amount less than the target ARA requirement amount; and DPA(n-6) in at least a threshold amount, wherein the ARA response curve is curvilinear, and wherein the threshold amount results in an ARA response at which the slope of the ARA response curve changes from negative to positive.

The invention also provides a method of preparing an infant formula composition, comprising combining nutritional components, DHA, ARA, and DPA(n-6), wherein DHA is present in a target DHA requirement amount; ARA is present in a sub-target amount less than a target ARA requirement amount; and DPA(n-6) is present in an amount sufficient to retroconvert to ARA *in vivo,* in an amount sufficient to offset the ARA sub-target amount. Infant formula compositions prepared by this method are also included in the invention.

The invention also provides a method of preparing an infant formula composition, comprising combining nutritional components, and a blend of microbial oils, wherein the blend is selected from the group consisting of a blend comprising a first oil comprising DHA and DPA(n-6) and a second oil comprising DHA; a blend comprising an oil having a first ratio of DHA and DPA(n-6) and second oil having a second DHA ratio DPA(n-6), wherein the ratios are different and wherein the first oil has more DPA(n-6) than the second oil; and a blend comprising a first oil from a thraustochytrid microorganism and a second oil from a dinoflagellate microorganism. Infant formula compositions prepared by this method are also included in the invention.

In certain aspects of these embodiments, the ARA sub-target amount is an amount selected from the group consisting of an amount about 5% below the target ARA requirement amount, an amount about 10% below the target ARA requirement amount, an amount about 15% below the target ARA requirement amount, and an amount about 20% below the target ARA requirement amount.

In certain aspects of these embodiments, the ratio of ARA:DHA is less than about 3:1, less than about 2.95:1, less than about 2.85:1, less than about 2.9:1, less than about 2.8:1, less than about 2.75:1, less, than about 2.5:1, less than about 2.25:1, less than about 2:1, less than about 1.95:1, less than about 1.9:1, less than about 1.85:1, less than about 1.8:1, or less than about 1.75:1. In other embodiments, the ratio of ARA:DHA is less than about 3:1, but greater than about 0.4:1: In still other embodiments, the composition does not comprise ARA.

In certain aspects of these embodiments, the long chain n-6 fatty acids in the infant formula compositions are supplied in a source oil, wherein the source oil comprises long chain n-6 fatty acids comprising at least about 2% by weight DPA(n-6). In other embodiments, the long chain n-6 fatty acids in the infant formula compositions are supplied in a source oil, wherein the source oil comprises long chain n-6 fatty acids comprising at least about 5% by weight DPA(n-6), at least about 10% by weight DPA(n-6), at least about 15% by weight DPA(n-6), at least about 20% by weight DPA(n-6), at least about 25% by weight DPA(n-6), at least about 30% by weight DPA(n-6), at least about 40% by weight DPA(n-6), or at least about 50% by weight DPA(n-6).

In certain aspects of these embodiments, the infant formula composition comprises at least 20 mg/L of DHA or at least 40 mg/L DHA. In other embodiments, the infant formula composition comprises from about 40 to about 140 mg/L of DHA, from about 20 to about 200 mg/L of DHA. In other embodiments, the composition comprises less than about 140 mg/L of DHA or less than about 200 mg/L of DHA.

In other aspects of these embodiments, the DHA:DPA(n-6) ratio is from about 5:1 1 to about 3:1, from about 5:1 to about 2:1, from about 5:1 to about 1:1, about 4:1, about 3:1, about 2:1, about 1:1, or about 0.5:1.

In certain aspects of these embodiments, the infant formula composition comprises at least about 7 mg/L of DPA(n-6), at least about 13 mg/L of DPA(n-6), at least about 26 mg/L of DPA(n-6), at least about at least about 40 mg/L of DPA(n-6), at least about 53 mg/L of DPA(n-6), or at least about 66 mg/L of DPA(n-6), at least about 80 mg/L of DPA(n-6), at least about 100 mg/L of DPA(n-6), at least about 120 mg/L of DPA(n-6). In other embodiments, the infant formula composition comprises less than about 240 mg/L of DPA(n-6), less than about 220 mg/L of DPA(n-6), or less than about 200 mg/L of DPA(n-6).

In certain aspects of these embodiments, the infant formula composition comprises from about 0.37 mg/L of cholesterol to about 500 mg/L cholesterol. In other embodiments, the infant formula composition comprises from about 0.37 mg/L of cholesterol to about 300 mg/L cholesterol, from about 0.37 mg/L of cholesterol to about 100 mg/L cholesterol, from about 0.37 mg/L of cholesterol to about 50 mg/L cholesterol, or from about 0.37 mg/L of cholesterol to about 10 mg/L cholesterol. In certain aspects of these embodiments, the infant formula composition comprises from about 0.37 mg/L of cholesterol to about 3.75 mg/L cholesterol. In some embodiments, the infant formula composition comprises greater than about 0.5 mg/L cholesterol. In some embodiments, the infant formula composition comprises about 0.37 mg/L cholesterol, about 0.75 mg/L cholesterol, about 1.5 mg/L cholesterol, about 2.3 mg/L cholesterol, about 3 mg/L cholesterol, or about 3.75 mg/L cholesterol. In certain aspects the cholesterol is provided in the form of a microbial oil, the oil containing a long chain omega-3 or omega-6 long chain fatty acid.

In certain aspects of these embodiments, the infant formula composition comprises eicosapentaenoic acid (EPA) in an amount less than about 3 mg/L. In other aspects, the infant formula composition comprises EPA in an amount less than about 60 mg/L. In other aspects, the infant formula composition comprises from about 15 mg/L to about 30 mg/L EPA. In some embodiments, the EPA:DHA ratio is provided in a ratio of up to 1: I.

In other aspects of these embodiments, the infant formula composition is formulated to provide at least about 2 mg/kg/day DPA(n-6) when administered to an infant. In some embodiments, the infant formula composition is formulated to provide from about 3 mg/kg/day DPA(n-6) to about 8 mg/kg/day DPA(n-6) when administered to an infant.

In some aspects of all of the foregoing embodiments, the DHA, ARA or DPA(n-6) is from a source selected from the group consisting of a plant, an oilseed, a microorganism, an animal, and mixtures of the foregoing. In some aspects, the microorganism is selected from the group consisting of algae, bacteria, fungi and protists. In some aspects, the microorganism selected from the group consisting of Thraustochytriales, dinoflagellates, and Mucorales. In other aspects, the microorganism is selected from the group consisting of *Schizochytrium, Thraustochytrium, Crypthecodinium,* and *Mortierella.*

In other aspects, the source is selected from the group consisting of genetically modified plant and genetically modified oilseed selected from the group consisting of soybean, corn, safflower, sunflower, canola, flax, peanut, mustard, rapeseed, chickpea, cotton, lentil, white clover, olive, palm, borage, evening primrose, linseed and tobacco and mixtures thereof.

In still other aspects, the source is selected from the group consisting of a genetically modified plant, a genetically modified oilseed, and a genetically modified microorganism, wherein the genetic modification comprises the introduction of a polyketide synthase gene or a portion thereof. As will be appreciated by one skilled in the art, a genetically modified oilseed is part of a genetically modified plant.

In further aspects, the composition comprises a blend of microbial oils, wherein the blend comprises a first oil comprising DHA and DPA(n-6) and a second oil comprising DHA.

In further aspects, the composition comprises a blend of microbial oils, wherein the blend comprises an oil having a first ratio of DHA and DPA(n-6) and second oil having a second ratio of DHA and DPA(n-6), wherein the ratios are different and wherein the first oil has more DPA(n-6) than the second oil.

In further aspects, the infant formula composition comprises a blend of microbial oils, and wherein the blend comprises oil from a thraustochytrid microorganism and oil from a dinoflagellate microorganism.

In further aspects, the composition comprises a blend of microbial and plant oils, wherein the blend comprises oil from a plant genetically modified to produce DHA and DPA(n-6), and wherein the plant oil comprises DHA and DPA(n-6), and wherein the microbial oil comprises ARA. In some aspects, the genetic modification comprises the introduction of a PUFA polyketide synthase gene or portions thereof.

In further aspects, the composition comprises a plant oil from a plant genetically modified to produce DHA and DPA(n-6), and wherein the oil comprises DHA and DPA(n-6) in a ratio of from about 0.5:1 to about 10:1. In further aspects, the composition comprises a plant oil from a plant genetically modified to produce one of DPA(n-6), DHA, or ARA. In preferred embodiments of this aspect, the microorganism does not produce one or both of the other two PUFAs. In some aspects, the genetic modification comprises the introduction of a PUFA polyketide synthase gene or portions thereof.

In further aspects, the composition comprises a microbial oil from a genetically modified microorganism and wherein the oil comprises DHA and DPA(n-6) in a ratio of from about 0.5:1 to about 10:1. In further aspects, the composition comprises a microbial oil from a microorganism genetically modified to produce one of DPA(n-6), DHA, or ARA. In preferred embodiments of this aspect, the microorganism does not produce one or both of the other two PUFAs. In some aspects, the genetic modification comprises the introduction of a PUFA polyketide synthase gene or portions thereof.

The invention further provides methods for feeding an infant comprising administering an infant nutritional composition of the invention to an infant.

The invention further provides a method feeding of an infant comprising administering an infant formula composition containing nutritional components, DHA and DPA(n-6) to an infant, wherein the infant is fed at least about 2 mg DPA(n-6)/kg/day.

As used herein, an amount/kg/day means the amount multiplied by the weight of the infant in kilograms, per day. As used herein, an amount/L means the amount in a liter of infant formula composition as intended for consumption by an infant, i.e., if the infant formula composition is manufactured as a dry powder or a concentrated liquid, the amount/L is measured when the dry powder or concentrated liquid has been mixed with sufficient liquid to achieve the infant formula composition intended to be consumed by an infant. When sources and amounts or ranges of the fatty acids and other ingredients are used herein, all combinations and subcombinations and specific embodiments therein are intended to be included.

### BRIEF DESCRIPTION OF THE DRAWINGS

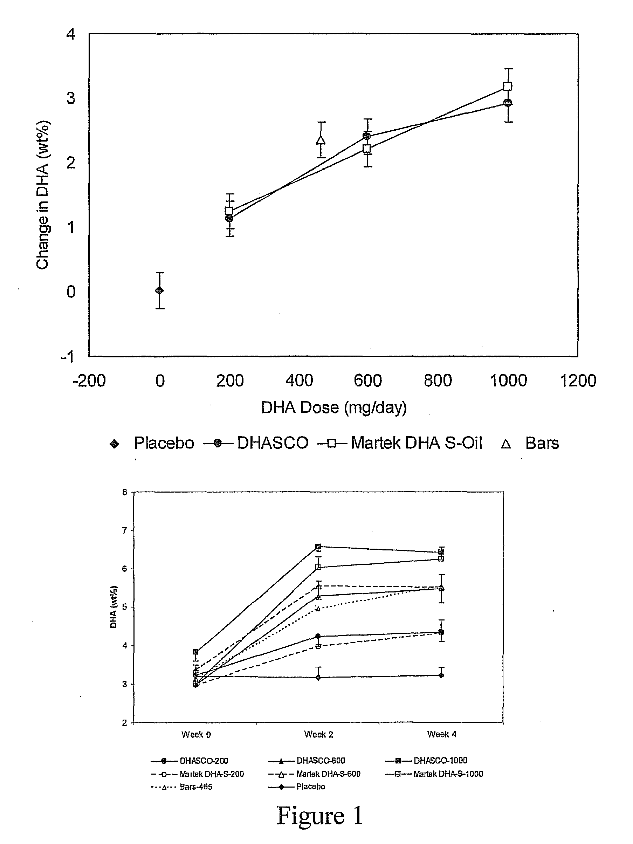

Figure 1 shows the 4-week change from baseline in plasma phospholipid (PL) DHA levels as weight percent of total fatty acids by DHA dose and DHA source for the DHA Bioavailability Study in Adults of Example 1 using DHA-rich *Schizochytrium* Martek DHA™-S Oil compared to a pure DHA oil (DHASCO®) (LSMean ± S.E., n = 12 individuals per data point).

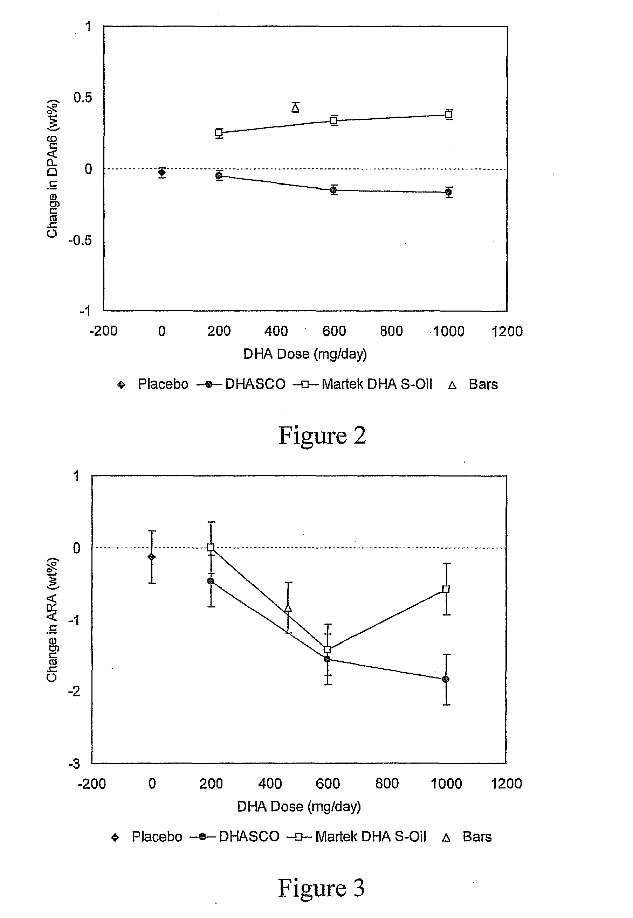

Figure 2 shows the 4-week change from baseline in plasma phospholipid (PL) DPA(n-6) levels as weight percent of total fatty acids by DHA dose and DHA source for the DHA Bioavailability Study in Adults of Example 1 using DHA-rich *Schizochytrium* Martek DHA™-S Oil compared to a pure DHA oil (DHASCO®) (LSMean ± S.E., n = 12 individuals per data point).
Figure 3 shows 4-week change from baseline levels in measured plasma phospholipid (PL) arachidonic acid levels as weight percent of total fatty acids by dose and DHA source for the DHA Bioavailability Study of Example 1 using DHA-rich *Schizochytrium* Martek DHA™-S Oil compared to a pure DHA oil (DHASCO®) (LSMean ± S.E., n =12 individuals per data point).

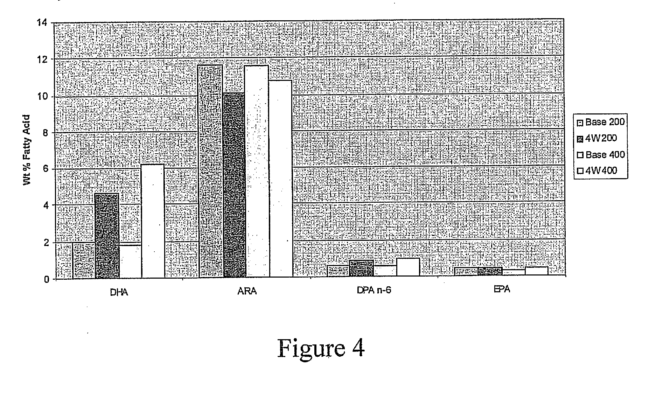

Figure 4 shows plasma phospholipid fatty acid levels as weight percent of total fatty acids for the DHA Bioavailability Study in Children of Example 2 before and after supplementation with 200 and 400 mg DHA/day with DHA-rich *Schizochytrium* Martek DHA™-S Oil for 4 weeks (n =10-12 individuals per group).

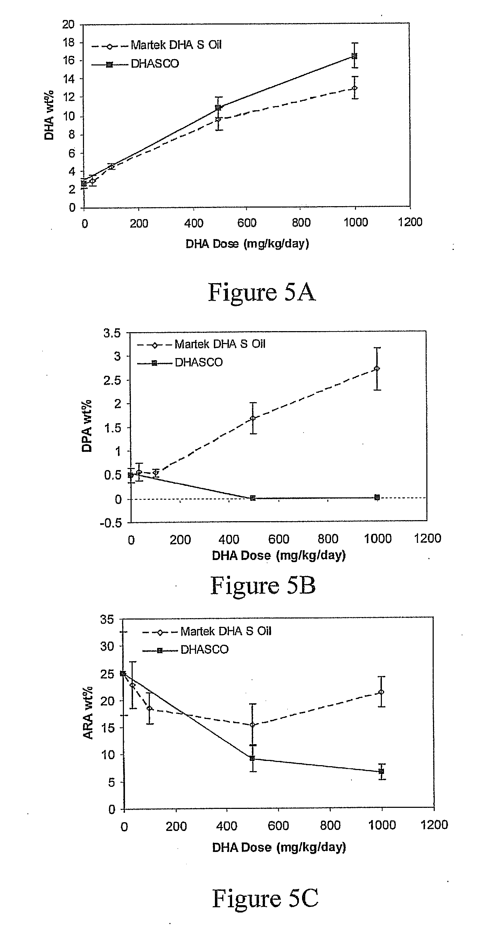

Figures 5A-C show DHA (A), DPA(n-6) (B) and ARA (C) levels in plasma following 28 days of feeding rats with a diet containing Martek DHA™-S oil or DHASCO® oil.

### DETAILED DESCRIPTION OF THE INVENTION

The present invention is directed to methods for preparing infant formula products and infant formula compositions that are supplemented with LC-PUFAs. Infant formula is defined by United States law as a food that purports to be or is represented for special dietary use solely as a food for infants by reason of its simulation of human milk or its suitability as a complete or partial substitute for human milk.

The infant formulas of the present invention in some embodiments can have ARA levels which are lower than current recommended levels or targets, but which nonetheless significantly reduce or prevent the associated decline in ARA plasma levels that will occur when the infant formula contains an omega-3 fatty acid such as DHA. Reducing or preventing the decline in ARA plasma levels maintains normal infant growth, weight, immune system development, and nervous system development.

As noted above, it has been suggested that LC-PLTFAs be included in infant formula in amounts that match or closely mimic the LC-PUFA profile found in human breast milk. The formulas of the present invention are based on the recognition that infant formula need not match or closely mimic the LC-PLTFA profile found in human breast milk in order to provide significant nutritional benefits. Specifically, the present inventors have discovered that the addition of DPA(n-6) to DHA supplemented infant formula, will help maintain plasma ARA levels in infants. Thus, the amount of ARA added to infant formula can be reduced to a sub-target amount to compensate for the ARA metabolized from DPA(n-6). As used herein, reference to ARA in a sub-target amount refers to an amount of ARA that is less than a target ARA requirement amount in an infant formula. An example of a target ARA requirement amount is the amount of ARA in human breast milk. This amount has been reported as 0.37-0.49% of total fatty acids in human breast milk. (Yuhas, et al., Lipids, 2006 41(9):851-58) Therefore, as used herein, one example of a target ARA requirement for an infant formula composition will be 0.37-0.49% of total fatty acids in the infant formula composition. Another example of a target ARA requirement amount is the amount of ARA added to an infant formula composition such that an approximate ratio of from about 2:1 to about 1:1 (ARA:DHA) is provided

The recognition of the inventors is the result of the discovery that supplementation with the DPA(n-6) results in a curvilinear ARA response, with reductions in ARA at low doses but a near maintenance of ARA levels at the higher doses. Thus, the present invention provides methods and compositions for infant formulas, in which at least some of the ARA in infant formula is replaced or supplemented by DPA(n-6): Without being bound by theory, it is believed that both DPA(n-6) and ARA are important as independent sources of eicosanoids which impact growth and for their ability to compete with LC ω-3 fatty acids such as DHA for incorporation into cell membranes. ARA can be elongated to DPA(n-6), and DPA(n-6) can be retroconverted to ARA. Without being bound by theory, the present inventors have found that at a threshold dose, DPA(n-6) actively retroconverts to ARA *in vivo,* a phenomenon that appears to be substrate-driven. For example, Figure 3 shows a curvilinear response of plasma ARA levels in which the slope of the ARA response changes from negative to positive, as amounts of DPA(n-6), a component of Martek DHA-S oil, are increased. Thus, Figure 3 illustrates a threshold dose of DPA(n-6). In addition, the present invention includes DHA supplemented infant formulas having conventional amounts of ARA supplementation, further including DPA(n-6) resulting in infant formulas that can achieve higher ARA (augmented) plasma levels due to the combination of the ARA in the formula and the retroconversion of DPA(n-6) to ARA.

Since the infant formulas of the invention comprise multiple components, including LC-PUFAs, it will be appreciated that the different embodiments of the infant formulas can be described by reference to one or more of the components, or ratios of such components. For example, the amount of ARA in the composition can be expressed as a ratio of ARA:DHA. The amount of DPA(n-6) in the composition can be expressed as ratio with DHA or as a concentration of DPA(n-6) contained in the formula, e.g., in a liter of the formula as intended for consumption by an infant. The infant formulas can also be described by using the amount of DPA(n-6) in the composition based on the ARA plasma levels that are desired; that is, DPA(n-6) in an amount sufficient to retroconvert to ARA *in vivo*, and sufficient to offset (compensate for) the amount of ARA in the formula that is present in a sub-target amount.

Accordingly, in one embodiment, the invention provides an infant formula composition wherein the ratio of ARA:DHA is less than about 3:1. In other embodiments, the ratio of ARA:DHA is less than about 2.95:1, less than about 2.85:1, less than about 2.9:1, less than about 2.8:1, less than about 2.75:1, less, than about 2.5:1, less than about 2.25:1, less than about 2:1, less than about 1.95:1, less than about 1.9:1, less than about 1.85:1, less than about 1.8:1, or less than about 1.75:1. In other embodiments, the ratio of ARA:DHA is greater than about 0.4:1. In still other embodiments, the composition does not comprise ARA.

In some embodiments, the DHA:DPA(n-6) ratio is from about 10:1 to about 0.5:1, from about 5:1 to about 0.5:1, from about 5:1 to about 3:1, from about 5:1 to about 2:1, from about 5:1 to about 1:1, about 4:1, about 3:1, about 2:1, about 1:1, or about 0.5:1.

In some embodiments, the long chain n-6 fatty acids in the infant formula compositions are supplied in a source oil, wherein the source oil comprises long chain n-6 fatty acids comprising at least about 2% by weight DPA(n-6). In other embodiments, the long chain n-6 fatty acids in the infant formula compositions are supplied in a source oil, wherein the source oil comprises long chain n-6 fatty acids comprising at least about 5% by weight DPA(n-6), at least about 10% by weight DPA(n-6), at least about 15% by weight DPA(n-6), at least about 20% by weight DPA(n-6), or at least about 25% by weight DPA(n-6), at least about 30% by weight DPA(n-6), at least about 40% by weight DPA(n-6), or at least about 50% by weight DPA(n-6).

In some embodiments, the composition comprises at least about 7 mg/L of DPA(n-6), at least about 13 mg/L of DPA(n-6), at least about 26 mg/L of DPA(n-6), at least about 40 mg/L of DPA(n-6), at least about 53 mg/L of DPA(n-6), or at least about 66 mg/L of DPA(n-6), at least about 80 mg/L of DPA(n-6), at least about 100 mg/L of DPA(n-6), at least about 120 mg/L of DPA(n-6). In other embodiments, the composition comprises less than about 240 mg/L of DPA(n-6), less than about 220 mg/L of DPA(n-6), or less than about 200 mg/L of DPA(n-6).

In some embodiments, the infant formula composition comprises at least 20 mg/L of DHA or at least 40 mg/L DHA. In other embodiments, the infant formula composition comprises from about 40 to about 140 mg/L of DHA, from about 20 to about 200 mg/L of DHA. In other embodiments, the composition comprises less than about 140 mg/L of DHA or less than about 200 mg/L of DHA.

In certain aspects of these embodiments, the infant formula composition comprises from about 0.37 mg/L of cholesterol to about 500 mg/L cholesterol. In other embodiments, the infant formula composition comprises from about 0.37 mg/L of cholesterol to about 300 mg/L cholesterol, from about 0.37 mg/L of cholesterol to about 100 mg/L cholesterol, from about 0.37 mg/L of cholesterol to about 50 mg/L cholesterol, or from about 0.37 mg/L of cholesterol to about 10 mg/L cholesterol. In other embodiments, the infant formula composition comprises from about 0.37 mg/L of cholesterol to about 3.75 mg/L cholesterol. In some embodiments, the infant formula composition comprises greater than about 0.5 mg/L cholesterol. In some embodiments, the infant formula composition comprises about 0.37 mg/L cholesterol, about 0.75 mg/L cholesterol, about 1.5 mg/L cholesterol, about 2.3 mg/L cholesterol, about 3 mg/L cholesterol, or about 3.75 mg/L cholesterol. In certain aspects the cholesterol is provided in the form of a microbial oil, the oil containing a long chain omega-3 or omega-6 long chain fatty acid.

In other embodiments, the infant formula composition comprises eicosapentaenoic acid (EPA) in an amount less than about 3 mg/L. In other aspects, the infant formula composition comprises EPA in an amount less than about 60 mg/L. In other aspects, the infant formula composition comprises from about 15 mg/L to about 30 mg/L EPA. In some embodiments, the EPA:DHA ratio is provided in a ratio of up to 1:1.

In other embodiments, the infant formula composition is formulated to provide at least about 2 mg/kg/day DPA(n-6) when administered to an infant. In some embodiments, the infant formula composition is formulated to provide from about 3 mg/lcglday DPA(n-6) to about 8 mg/kg/day DPA(n-6) when administered to an infant.

In some embodiments, an ARA sub-target amount is an amount selected from the group consisting of an amount about 5% below a target ARA requirement amount, an amount about 10% below a target ARA requirement amount, an amount about 15% below a target ARA requirement amount, and an amount about 20% below a target ARA requirement amount.

In one embodiment, the invention provides an infant formula composition, wherein the composition comprises long chain n-3 fatty acids and long chain n-6 fatty acids, and in which the long chain n-3 fatty acids comprise docosahexaenoic acid (DHA); the long chain n-6 fatty acids comprise docosapentaenoic acid (DPA(n-6)) and optionally arachidonic acid (ARA). In this embodiment, the ratio of ARA:DHA is less than about 3:1, and the DHA:DPA(n-6) ratio is from about 5:1 to about 0.5:1.

The invention also provides a method of preparing an infant formula composition, comprising combining nutritional components, long chain n-3 fatty acids and long chain n-6 fatty acids; wherein the long chain n-3 fatty acids comprise DHA; wherein the long chain n-6 fatty acids comprise ARA and DPA(n-6); wherein the ratio of AR.A.:DHA is less than about 3:1, and wherein the DHA:DPA(n-6) ratio is from about 5:1 to about 0.5:1. Infant formula compositions prepared by this method are also included in the invention.

In another embodiment, the infant formula comprises long chain n-6 fatty acids and DHA, in which the long chain n-6 fatty acids in the infant formula compositions are supplied in a source oil, wherein the source oil comprises long chain n-6 fatty acids comprising at least about 2% by weight DPA(n-6), the DHA:DPA(n-6) ratio is from about 5:1 to about 0.5:1, and wherein the composition is formulated to provide at least about 2 mg/kg/day DPA(n-6) when administered to an infant:

The invention also provides a method of preparing an infant formula composition, comprising combining nutritional components, DHA, and the long chain n-6 fatty acids, wherein the long chain n-6 fatty acids in the infant formula compositions are supplied in a source oil, wherein the source oil comprises long chain n-6 fatty acids comprising at least about 2% by weight DPA(n-6), wherein the DHA:DPA(n-6) ratio is from about 5:1 to about 0.5:1. The method is designed to provide a formulation that provides at least about 2 mg/kg/day DPA(n-6) when administered to an infant. Infant formula compositions prepared by this method are also included in the invention.

In another embodiment, the infant formula composition is formulated to comprise at least about 7 mg/L of DPA(n-6) when ready for consumption, wherein the composition further comprises long chain n-3 fatty acids; wherein the long chain n-3 fatty acids comprise DHA; wherein the composition further optionally comprises arachidonic acid ARA; and wherein the ratio of ARA:DHA is less than about 3:I .

The invention also provides a method of preparing an infant formula composition, comprising combining nutritional components, DPA(n-6), DHA, and optionally, ARA; wherein the composition is formulated to comprise at least about 7 mg/L of DPA(n-6) when ready for consumption, and wherein the ratio of ARA:DHA is less than about 3:1. Infant formula compositions prepared by this method are also included in the invention.

In yet another embodiment the invention provides an infant formula composition formulated to meet DHA and ARA target requirements, and the composition comprises DHA in the target DHA requirement amount, ARA in a sub-target amount less than the target ARA requirement amount; and DPA(n-6) in an amount sufficient to retroconvert to ARA *in vivo,* in an amount sufficient to offset the ARA sub-target amount. It should be noted that infants are not considered to have a physiological deficiency in the present invention (e.g., infants do not have an ARA deficiency). Rather, the term "sub-target" is used herein to refer to an amount that is less than a recommended or target amount. For example, a widely recommended or target amount of ARA is an amount sufficient to provide an ARA:DHA ratio of 2:1, such that appropriate plasma levels of ARA and DHA are achieved.

A method of preparing an infant formula composition, comprising combining nutritional components, DHA, ARA, and DPA(n-6), wherein DHA is present in a target DHA requirement amount; ARA is present in a sub-target amount less than a target ARA requirement amount; and DPA(n-6) is present in an amount sufficient to retroconvert to ARA *in vivo,* in an amount sufficient to offset the ARA sub-target amount. Infant formula compositions prepared by this method are also included in the invention.

The ARA, DHA, and DPA(n-5), and other PUFAs referred to herein, such as EPA, can be in any of the common forms found in natural lipids including but not limited to triacylglycerols, diacylglycerols, monoacylglycerols, phospholipids, free fatty acids, esterified fatty acids, or in natural or synthetic derivative forms of these fatty acids (e.g. calcium salts of fatty acids, ethyl esters, etc). Reference to an oil comprising a PUFA, as used in the present invention, can refer to either an oil comprising only a single PUFA such as DHA or an oil comprising a mixture of two or more PUFAs such as DHA and EPA, or DHA and DPA(n-6).

A preferred source of an oil comprising at least one PUFA, in the compositions and methods of the present invention, includes a microbial source. Microbial sources and methods for growing microorganisms comprising nutrients and/or PUFAs are known in the art (Industrial Microbiology and Biotechnology, 2nd edition, 1999, American Society for Microbiology). Preferably, the microorganisms are cultured in a fermentation medium in a fermentor. The methods and compositions of the present invention are applicable to any industrial microorganism that produces any kind of nutrient or desired component such as, for example algae, protists, bacteria and fungi (including yeast).

Microbial sources can include a microorganism such as an algae, bacteria, fungi and/or protist. Preferred organisms include those selected from the group consisting of golden algae (such as microorganisms of the kingdom Stramenopiles), green algae, diatoms, dinoflagellates (such as microorganisms of the order Dinophyceae including members of the genus *Crypthecodinium* such as, for example, *Crypthecodinium cohnii*), yeast, and fungi of the genera *Mucor* and *Mortierella,* including but not limited to *Mortierella alpina* and *Mortierella* sect. *schmuckeri.* Members of the microbial group Stramenopiles include microalgae and algae-like microorganisms, including the following groups of microorganisms: Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales. The Thraustochytrids include the genera *Schizochytrium* (species include *aggregatum, limnaceum, mangrovei, minutum, octosporum*), *Thraustochytrium* (species include *arudimentale, aureum, benthicola, globosum, kinnei, motivum, multirudimentale, pachydermum, proliferum, roseum, striatum), Ulkenia** (species include *amoeboidea, kerguelensis, minuta, profunda, radiate, sailens, sarkariana, schizochytrops, visurgensis, yorkensis*), *Aplanochytrium* (species include *haliotidis, kerguelensis, profunda, stocchinoi*), *Japonochytrium* (species include *marinum*), *Althornia* (species include *crouchii*), and *Elina* (species include *marisalba, sinorifica*). Since there is some disagreement among experts as to whether *Ulkenia* is a separate genus from the genus *Thraustochytrium,* for the purposes of this application, the genus *Thraustochytrium* will include *Ulkenia.* The Labrinthulids include the genera *La byrinthula* (species include *algeriensis, coenocystis, chattonii, macrocystis, macrocystis atlantica, macrocystis macrocystis, marina, minuta, roscoffensis, valkanovii, vitellina, vitellina pacifica, vitellina vitellina, zopfi*), *Labyrinthomyxa* (species include *marina*), *Labyrinthuloides* (species include *haliotidis, yorkensis*), *Diplophrys* (species include *archeri*), *Pyrrhosorus** (species include *marinus*), Sorodiplophrys* (species include *stercorea*), Chlamydomyxa* (species include *labyrinthuloides, montana*). (* = there is no current general consensus on the exact taxonomic placement of these genera).

While processes of the present invention can be used to produce forms of PUFAs that can be produced in a wide variety of microorganisms, for the sake of brevity, convenience and illustration, this detailed description of the invention will discuss processes for growing microorganisms which are capable of producing lipids comprising omega-3 and/or omega-6 polyunsaturated fatty acids, in particular microorganisms that are capable of producing DHA (or closely related compounds such as DPA, EPA or ARA). Additional preferred microorganisms are algae, such as Thraustochytrids of the order Thraustochytriales, including *Thraustochytrium* (including *Ulkenia*), and *Schizochytrium,* and including Thraustachytriales which are disclosed in commonly assigned U.S. Patent Nos. 5,340,594 and 5,340,742, both issued to Barclay, all of which are incorporated herein by reference in their entirety. More preferably, the microorganisms are selected from the group consisting of microorganisms having the identifying characteristics of ATCC number 20888, ATCC number 20889, ATCC number 20890, ATCC number 20891 and ATCC number 20892. Also preferred are strains of *Mortierella schmuckeri* (e.g., including microorganisms having the identifying characteristics of ATCC 74371) and *Mortierella alpina* (e.g., including microorganisms having the identifying characteristics of ATCC 42430). Also preferred are strains of *Crypthecodinium cohnii,* including microorganisms having the identifying characteristics of ATCC Nos. 30021, 30334-30348, 30541-30543, 30555-30557, 30571, 30572, 30772-30775, 30812, 40750, 50050-50060, and 50297-50300. Also preferred are mutant strains derived from any of the foregoing, and mixtures thereof. Oleaginous microorganisms are also preferred. As used herein, "oleaginous microorganisms" are defined as microorganisms capable of accumulating greater than 20% of the weight of their cells in the form of lipids. Genetically modified microorganisms that produce PUFAs are also suitable for the present invention. These can include naturally PUFA-producing microorganisms that have been genetically modified as well as microorganisms that do not naturally produce PUFAs but that have been genetically modified to do so.

Suitable organisms may be obtained from a number of available sources, including by collection from the natural environment. For example, the American Type Culture Collection currently lists many publicly available strains of microorganisms identified above. As used herein, any organism, or any specific type of organism, includes wild strains, mutants, or recombinant types. Growth conditions in which to culture or grow these organisms are known in the art, and appropriate growth conditions for at least some of these organisms are disclosed in, for example, U.S. Patent No. 5,130,242, U.S. Patent No. 5,407,957, U.S. Patent No. 5,397,591, U.S. Patent No. 5,492,938, and U.S. Patent No. 5,711,983, all of which are incorporated herein by reference in their entirety.

Another preferred source of an oil comprising at least one PUFA, in the compositions and methods of the present invention includes a plant source, such as oilseed plants. Since plants do not naturally produce PUFAs having carbon chains of 20 or greater, plants producing such PUFAs are those genetically engineered to express genes that produce such PUFAs. As described in copending U.S. Patent Application Ser. No. 11/452,096,entitled, PUFA polyketide synthase systems and uses thereof, and filed June 12, 2006, which is incorporated herein by reference in its entirety, PUFA PKS systems represent an opportunity to produce plants with unique fatty acid production capabilities, such as plants genetically engineered to produce one or more PUFAs in the same plant, including, DHA and DPA(n-6). The creation any one of a number of "designer oils" in various ratios and forms is also disclosed.

In some embodiments, the oil comprising at least one PUFA is a plant seed oil derived from an oil seed plant that has been genetically modified to produce long chain polyunsaturated fatty acids. Such genes can include genes encoding proteins involved in the classical fatty acid synthase pathways, or genes encoding proteins involved in the PITFA polyketide synthase (PKS) pathway. The genes and proteins involved in the classical fatty acid synthase pathways, and genetically modified organisms, such as plants, transformed with such genes, are described, for example, in Napier and Sayanova, Proceedings of the Nutrition Society (2005), 64:387-393; Robert et al., Functional Plant Biology (2005) 32:473-479; or U.S. Patent Application Publication 2004/0172682. The PUFA PKS pathway, genes and proteins included in this pathway, and genetically modified microorganisms and plants transformed with such genes for the expression and production of PUFAs are described in detail in: U.S. Patent No. 6,566,583; U.S. Patent No. 7,247,461; U.S. Patent No. 7,211,418; and U.S. Patent No. 7,217,856, each of which is incorporated herein by reference in its entirety.

Preferred oilseed crops include soybeans, corn, safflower, sunflower, canola, flax, peanut, mustard, rapeseed, chickpea, cotton, lentil, white clover, olive, palm oil, borage, evening primrose, linseed, and tobacco that have been genetically modified to produce PUFA as described above.

Genetic transformation techniques for microorganisms and plants are well-known in the art. Transformation techniques for microorganisms are well known in the art and are discussed, for example, in Sambrook et al., 1989, Molecular Cloning: A Laboratory Manual, Cold Spring Harbor Labs Press. A general technique for transformation of dinoflagellates, which can be adapted for use with *Crypthecodinium cohnii,* is described in detail in Lohuis and Miller, The Plant Journal (1998) 13(3): 427-435. A general technique for genetic transformation of Thraustochytrids is described in detail in U.S. Patent Application Publication No. 20030166207, published September 4, 2003. Methods for the genetic engineering of plants are also well known in the art. For instance, numerous methods for plant transformation have been developed, including biological and physical transformation protocols. See, for example, Miki et al., "Procedures for Introducing Foreign DNA into Plants" in Methods in Plant Molecular Biology and Biotechnology, Glick, B.R. and Thompson, J.E. Eds. (CRC Press, Inc., Boca Raton, 1993) pp. 67-88. In addition, vectors and *in vitro* culture methods for plant cell or tissue transformation and regeneration of plants are available. See, for example, Gruber et al., "Vectors for Plant Transformation" in Methods in Plant Molecular Biology and Biotechnology, Glick, B.R. and Thompson, J.E. Eds. (CRC Press, Inc., Boca Raton, 1993) pp. 89-119. See also, Horsch et al., Science 227:1229 (1985); Kado, C.I., Crit. Rev. Plant. Sci. 10:1 (1991); Moloney et al., Plant Cell Reports 8:238 (1989); U.S. Patent No. 4,940,838; U.S. Patent No. 5,464,763; Sanford et al., Part. Sci. Technol. 5:27 (1987); Sanford, J.C., Trends Biotech. 6:299 (1988); Sanford, J.C., Physiol. Plant 79:206 (1990); Klein et al., Biotechnology 10:268 (1992); Zhang et al., Bio/Technology 9:996 (1991); Deshayes et al., EMBO J., 4:2731 (1985); Christou et al., Proc Natal. Acad. Sci. USA 84:3962 (1987); Hain et al., Mol. Gen. Genet. 199:161 (1985); Draper et al., Plant Cell Physiol. 23:451 (1982); Donn et al., In Abstracts of VIIth International Congress on Plant Cell and Tissue Culture IAPTC, A2-38, p. 53 (1990); D'Halluin et al., Plant Cell 4:1495-1505 (1992) and Spencer et al., Plant Mol. Biol. 24:51-61 (1994).

When oilseed plants are the source of PUFA, the seeds can be harvested and processed to remove any impurities, debris or indigestible portions from the harvested seeds. Processing steps vary depending on the type of oilseed and are known in the art. Processing steps can include threshing (such as, for example, when soybean seeds are separated from the pods), dehulling (removing the dry outer covering, or husk, of a fruit, seed, or nut), drying, cleaning, grinding, milling and flaking. After the seeds have been processed to remove any impurities, debris or indigestible materials, they can be added to an aqueous solution, generally, water and then mixed to produce a slurry. Generally, milling, crushing or flaking is performed when ready for mixing with water. A slurry produced in this manner can be treated and processed the same way as described for a microbial fermentation broth. Size reduction, heat treatment, pH adjustment, pasteurization and other known treatments can be used in order to improve hydrolysis, emulsion preparation, and quality (nutritional and sensory).

Another preferred source of an oil comprising at least one PUFA, in the compositions and methods of the present invention includes an animal source. Thus, in some embodiments, the oil comprising at least one PUFA is an aquatic animal oil. Examples of animal sources include aquatic animals (e.g., fish, marine mammals, and crustaceans such as krill and other euphausids) and lipids extracted from animal tissues (e.g., brain, liver, eyes, etc.) and animal products such as eggs or milk.

The invention further provides infant formula comprising blends of microbial oils, blends of microbial and plant oils, plant oils from plants genetically modified to produce DHA and DPA(n-6), or microbial oils from microorganisms which are genetically modified.

In one embodiment, the invention provides an infant formula composition comprising a blend of microbial oils, in which the blend comprises oil from a thraustochytrid microorganism and oil from a dinoflagellate microorganism.

In one embodiment, the thraustochytrid microorganism is of the genus *Schizochytrium* and the dinoflagellate microorganism is of the genus *Crypthecodinium.*

In another embodiment, the blend comprises between about 20 wt. % and about 90 wt. %, between about 50 wt. % and about 82 wt. %, or about 75 wt. % oil from a thraustochytrid microorganism and between about 10 wt. % and about 80 wt. %, between about 18 wt. % and about 50 wt. %, or about 25 wt. % oil from a dinoflagellate microorganism. In another embodiment, the blend comprises between about 57.5% by weight and about 67.5% by weight oil from a thraustochytrid microorganism and between about 32.5% by weight and about 42.5% by weight oil from a dinoflagellate microorganism. In another embodiment, the blend comprises between about 65% by weight and about 80% by weight oil from a thraustochytrid microorganism and between about 35% by weight and about 20% by weight oil from a dinoflagellate microorganism. In another embodiment, the blend comprises between about 80% by weight and about 99% by weight oil from a thraustochytrid microorganism and between about 20% by weight and about 1% by weight oil from a dinoflagellate microorganism.

In another embodiment, the invention provides an infant formula composition comprising a blend of microbial and plant oils. In this embodiment, the blend comprises oil from a plant genetically modified to produce DHA and DPA(n-6). The plant oil contains DHA and DPA(n-6), and the microbial oil contains ARA.

In another embodiment, the invention provides an infant formula composition comprising a plant oil from a plant genetically modified to produce DHA and DPA(n-6). The oil comprises DHA and DPA(n-6) in a ratio of from about 0.5:1 to about 5:1. In some embodiments, the plant is genetically modified to produce ARA.

Production of PUFAs (such as DHA, DPA(n-6), and ARA) in a plant that has been genetically modified to express the genes encoding a PUPA PKS system from *Schizochytrium* has been described in copending U.S. Patent Application Ser. No. 11/452,096, entitled, PUFA polyketide synthase systems and uses thereof, and filed June 12, 2006, which is incorporated herein by reference in its entirety. Briefly, the oils produced by these plants contain significant quantities of both DHA (docosahexaenoic acid (C22:6n-3)) and DPA (docosapentaenoic acid (C22:5n-6), which are the predominant PUFAs (the primary PUFAs) produced by the *Schizochytrium* from which the PUFA PKS genes were derived. Significantly, oils from plants that produce PUFAs using the PUFA PKS pathway have a different fatty acid profile than plants that are genetically engineered to produce the same PUFAs by the "classical" pathway. (In the classical pathway, medium chain-length saturated fatty acids (products of a fatty acid synthase (FAS) system) are modified by a series of elongation and desaturation reactions. The substrates for the elongation reaction are fatty acyl-CoA (the fatty acid chain to be elongated) and malonyl-CoA (the source of the 2 carbons added during each elongation reaction). The product of the elongase reaction is a fatty acyl-CoA that has two additional carbons in the linear chain. The desaturases create *cis* double bonds in the preexisting fatty acid chain by extraction of 2 hydrogens in an oxygen-dependant reaction. Such pathways and the genes involved in such pathways are well-known in the literature.) The use of PUFA PKS constructs to produce PUFAs in yeast, in addition to the production of the transgenic plants, is also disclosed in U.S. Patent Application Ser. No. 11/452,096. It was demonstrated that transformation of both yeast and plants with a PUFA PKS system that produces DHA and DPA(n-6) as the target PUFAs produces both of these PUFAs as the primary additional fatty acids in the total fatty acids of the plant (i.e., subtracting fatty acids that are produced in the wild-type plant), and in the yeast and further, that any other fatty acids that are not present in the fatty acids of the wild-type plant are virtually undetectable. Also disclosed is a genetically modified plant that produces DHA and DPA(n-6), and the ratio of DHA to DPA(n-6) is from about 1:10 to about 10:1, including any ratio in between.

In another embodiment, the invention provides an infant formula composition comprising a microbial oil from a genetically modified microorganism. The oil comprises DHA and DPA(n-6) in a ratio of from about 0.5:1 to about 5:1. In some embodiments, the microorganism produces ARA.

In another embodiment, the composition comprises a plant oil or microbial oil from a plant or microorganism genetically modified to produce DPA(n-6). In preferred embodiments of this aspect, the plant or microorganism does not produce DHA or ARA. In further aspects, the composition comprises a plant or microbial oil from a plant or microorganism genetically modified to produce DHA. In preferred embodiments of this aspect, the plant or microorganism does not produce DPA(n-6) or ARA. In further aspects, the composition comprises a plant or microbial oil from a microorganism genetically modified to produce ARA. In preferred embodiments of this aspect, the plant or microorganism does not produce DHA or DPA(n6). In one embodiment, oils from these plants or microorganisms are used in appropriate amounts and ratios to produce the infant formula compositions of the present invention.

In embodiments in which a genetically modified plant is utilized, the genetic modification can include the introduction of a PUFA polyketide synthase gene or portions thereof.

In some embodiments in which a genetically modified microorganism is utilized, the genetic modification can include the introduction of a PUFA polyketide synthase gene or portions thereof.

Nutritional components of infant formulas are known in the art and one knowledgeable in the art would be able to adjust formula compositions to include PUFA levels and ratios of the instant invention. For example, an infant formula typically contains a protein component comprising from about 6 to about 25% of the total caloric content of the infant formula; a carbohydrate component comprising from about 35 to about 50% of the total caloric content of the infant formula; and a lipid component comprising from about 30 to about 50% of the total caloric content of the infant formula. These ranges are provided as examples only, and are not intended to be limiting.

Examples of suitable fat sources typically include high oleic safflower oil, soy oil, fractionated coconut oil (medium chain triglycerides, MCT oil), high oleic sunflower oil, corn oil, canola oil, coconut, palm and palm kernel oils, marine oil, cottonseed oil, walnut oil, wheat germ oil, sesame oil, cod liver oil, and peanut oil. Any single fat listed above, or any combination thereof, as appropriate may be utilized. Other suitable fats will be readily apparent to those skilled in the art.

Additional components of infant formula typically include, for example, protein, carbohydrates, vitamins and minerals. Examples of suitable protein sources for an infant typically include casein, whey, condensed skim milk, nonfat milk, soy, pea, rice, corn, hydrolyzed protein, free amino acids, protein sources which contain calcium in a colloidal suspension with the protein. Any single protein listed above, or any combination thereof, as appropriate may be utilized. Other suitable proteins will be readily apparent to those skilled in the art.

A third component of infant formula is a source of carbohydrates. Carbohydrates are a major source of readily available energy that the infant needs for growth and that protects the infant from tissue catabolism. In human milk and most standard milk-based infant formulas, the carbohydrate is lactose. The carbohydrates that may be used in the infant formula can vary widely. Examples of carbohydrates suitable for infants typically include hydrolyzed cornstarch, maltodextrin, glucose polymers, sucrose, lactose, corn syrup, corn syrup solids, rice syrup, glucose, fructose, high fructose corn syrup and indigestible oligosaccharides such as frurtooligosaccharides (FOS). Any single carbohydrate listed above, or any combination thereof, as appropriate may be utilized. Other suitable carbohydrates will be readily apparent to those skilled in the art.

The infant formula of the present invention typically includes supplemented vitamins and minerals. Examples of vitamins and minerals that may be added to the infant formula of the instant invention typically include vitamin A, vitamin B₁, vitamin B₂, vitamin B₆, vitamin B₁₂, vitamin C, vitamin D, vitamin K, vitamin E, biotin, folic acid, pantothenic acid, niacin, m-inositol, calcium, phosphorus, magnesium, zinc, manganese, copper, sodium, potassium, chloride, iron and selenium. The additional nutrients chromium, molybdenum, iodine, taurine, carnitine and choline may also be included.

The infant formulas of the present invention may be prepared as any product form suitable for use in infants, including reconstitutable powders, ready-to-feed liquids, and dilutable liquid concentrates, which product forms are all well known in the nutritional formula art. As used in the present application, the amounts of components present in infant formula compositions refer to the amounts when the formula is ready for consumption by the infant. It is to be understood that in the case of a reconstitutable powder or dilutable liquid concentrate, the component amounts will be adjusted such that when the infant formula composition is reconstituted or diluted the amounts are as described herein. Thus, for example, reference to a infant formula composition that is to be diluted by, for example, addition of one part water for one part infant formula, wherein the infant formula composition has a given component concentration, when ready for consumption, is intended to cover an infant formula composition having a concentration of the component of twice the given amount, before it is made ready for consumption by the addition of water. Methods to prepare infant formulas are known to those skilled in the art. For example, the PUFA-containing oils can be added directly to a liquid formula composition at a suitable point in the manufacturing process.

The infant formula according to the present invention can optionally be sterilized and subsequently used on a ready-to-feed basis, or can be stored as a concentrate. The concentrate can be prepared by spray drying the liquid formula prepared as above, and the formula can be reconstituted by rehydrating the concentrate. The infant formula concentrate is a stable liquid and has a suitable shelf life.

In another embodiment, the oils are microencapsulated prior to the addition into a formula composition. The choice of coating for the microencapsulation of the PUFAs is determined by its lack of toxicity, desired particle size, and stability under the processing conditions for infant formulas, particularly sterilization. Any conventionally acceptable substantially oxygen-impermeable coating can be used in the present invention. Such conventional microencapsulating methods and coating materials are well within the purview of one skilled in the art, and the specific microencapsulating method and coating are not peculiar to the present invention. Some of these methods include spray drying such as where the PUFAs are emulsified into a solution of a polymer, and spray-dried to make fine particles. Particles of about 250 µm are suitable for inclusion in the infant formulas according to the present invention. When the PUFAs are incorporated into a meltable fat or wax, the process is called spray-chilling, since the emulsion need only be chilled below its melting point to form particles. Another encapsulation process that can be used to encapsulate the PUFAs is coacervation. Other suitable techniques include interfacial polymerization, hot melt encapsulation, phase separation encapsulation (solvent removal and solvent evaporation), spontaneous emulsion, solvent evaporation microencapsulation, solvent removal microencapsulation, coacervation, and low temperature microsphere formation and phase inversion nanoencapsulation (PIN). In general, the microencapsulated PUFAs form a free-flowing powder which is suitable for addition into powdered embodiments of the compositions. These and other encapsulation techniques and encapsulated oils are described in United States Provisional Patent Application Ser. No. 60/805,590, which is incorporated by reference herein in its entirety.

For powder embodiments of the present invention, the above-described methods of use further comprise reconstitution of the powder with a suitable aqueous liquid, preferably water. Such dilution may be in an amount sufficient to provide an LC-PUFA fortified infant formula having the characteristics described in detail herein. For powder embodiments of the present invention, such powders are typically in the form of flowable or substantially flowable particulate compositions, or at least particulate compositions that can be easily scooped and measured with a spoon or similar other device, wherein the compositions can easily be reconstituted by the intended user with a suitable aqueous fluid, typically water, to form a liquid infant formula. In this context, "immediate" use generally means within about 48 hours, most typically within about 24 hours, preferably right after reconstitution. These powder embodiments include spray dried, agglomerated, dry mixed or other known or otherwise effective particulate form. The quantity of a nutritional powder required to produce a volume suitable for one serving can vary.

The invention further provides methods for feeding an infant comprising administering an infant nutritional composition of the invention to an infant. For example, the invention provides a method of feeding an infant comprising administering an infant formula composition to an infant, wherein the composition comprises long chain n-3 fatty acids and long chain n-6 fatty acids, and in which the long chain n-3 fatty acids comprise docosahexaenoic acid (DHA); the long chain n-6 fatty acids comprise docosapentaenoic acid (DPA(n-6)) and optionally arachidonic acid (ARA). In this embodiment, the ratio of ARA:DHA is less than about 3:1, and the DHA:DPA(n-6) ratio is from about 5:1 to about 0.5:1. In another embodiment, the invention provides a method of feeding an infant comprising administering an infant formula composition to an infant, wherein the composition comprises long chain n-6 fatty acids and DHA, in which the long chain n-6 fatty acids in the infant formula compositions are supplied in a source oil, wherein the source oil comprises long chain n-6 fatty acids comprising at least about 2% by weight DPA(n-6), the DHA:DPA(n-6) ratio is from about 5:1 to about 0.5:1, and wherein the composition is formulated to provide at least about 2 mg/kg/day DPA(n-6) when administered to an infant. In another embodiment, the invention provides a method of feeding an infant comprising administering an infant formula composition to an infant, wherein the composition comprises at least about 7 mg/L of DPA(n-6) when ready for consumption, wherein the composition further comprises long chain n-3 fatty acids; wherein the long chain n-3 fatty acids comprise DHA; wherein the composition further optionally comprises arachidonic acid ARA; and wherein the ratio of ARA:DHA is less than about 3:1. In another embodiment, the invention provides a method of feeding an infant comprising administering an infant formula composition to an infant, wherein the infant formula composition is formulated to meet DHA and ARA target requirements, and the composition comprises DHA in the target DHA requirement amount, ARA in a sub-target amount less than the target ARA requirement amount; and DPA(n-6) in an amount sufficient to retroconvert to ARA *in vivo*, in an amount sufficient to offset the ARA sub-target amount.

The invention further provides a method of feeding an infant comprising administering an infant formula composition containing nutritional components, DHA and DPA(n-6) to an infant, wherein the infant is fed at least about 2 mg DPA(n-6)/kg/day. In some embodiments, the present invention provides a method of feeding an infant comprising administering any infant formula composition as described herein to an infant. In some embodiments, the method further comprises dissolving an infant formula powder containing nutritional components and DHA and DPA(n-6) in water for obtaining a solution including said nutritional components and said LC-PUFAs.

The nutritional formulas of the present invention may be packaged and sealed in single or multi-use containers, and then stored under ambient conditions for up to about 36 months or longer, more typically from about 12 to about 24 months. For multi-use containers, these packages can be opened and then covered for repeated use by the ultimate user, provided that the covered package is then stored under ambient conditions (e.g., avoid extreme temperatures) and the contents used within about one month or so.

Premature infants require additional nutrients to support their growth and are at risk for the diseases related to prematurity. Preterm infants are commonly fed either a commercial infant formula designed specifically for these infants or their own mother's milk. Another means of feeding a preterm infant is to supplement preterm milk, banked term milk, other suitable milk, or infant formula with a milk or formula fortifier. Such supplemented milk or formula can more adequately provide levels of several nutrients to meet the needs of these infants. Another invention of this application provides a premature infant nutrition fortifier composition comprising LC-PUFAs. In particular, the premature infant nutrition fortifier composition comprises DPA(n-6) and in other embodiments, can comprise DHA and/or ARA. The fortifier composition is generally a powder or oil which can optionally supplements level of protein, fat, vitamins and minerals. The fortifier compositions are formulated to provide the amounts and ratios of LC-PUFAs as described for infant formula compositions above, when added to milk or formula. For example, a fortified milk or formula composition can comprise a threshold amount of DPA(n-6), can have a DHA:DPA(n-6) ratio that is from about 5:1 to about 0.5:1, or have other limitations from the various inventions described herein. The fortifer compositions can be the sole source of one or more of the DPA(n-6), DHA and/or ARA LC-PUFAs in the resulting fortified milk or formula or can supplement amounts of LC PUFAs in the unfortified milk or formula. Another invention of the present application is a method of preparing a fortified premature infant milk or formula that includes combining an infant milk or formula with the composition as described.

Another invention of the present application is a method of providing supplemental nutrients to a preterm infant comprising administering a fortified milk or formula to a premature infant wherein the milk or formula is prepared by adding a premature infant nutrition fortifier composition as described above to an infant milk or formula.

Another invention of the present application is a method for promoting growth of a premature infant comprising administering a fortified milk or formula to a premature infant wherein the milk or formula is prepared by adding a premature infant nutrition fortifier composition as described above to an infant milk or formula.

The present invention also provides an infant dietary supplement composition supplemented with LC-PUFAs. In particular, the infant dietary supplement composition comprises DPA(n-6) and in other embodiments, can comprise DHA and/or ARA. The infant dietary supplement can also optionally supplement levels of protein, fat, vitamins and minerals. The infant dietary supplement compositions are formulated to provide the amounts and ratios of LC-PUFAs as described for infant formula compositions above, particularly including the daily intake parameters when such supplements are taken apart from infant formula. The infant dietary supplements, in some embodiments, however, can be added to infant formula. The infant dietary supplement of the present inventive subject matter may be formulated to be administered or are administered in a partial, *i.e.,* fractional dose, one or more times during a 24 hour period, a single dose during a 24 hour period of time, a double dose during a 24 hour period of time, or more than a double dose during a 24 hour period of time. Fractional, double or other multiple doses may be taken simultaneously or at different times during the 24 hour period.

Compositions for oral formulations useful for the infant dietary supplement composition invention that are palatable to infants are known in the art. The infant dietary supplement composition can be orally administered, for example, with an inert diluent or with an assimilable edible carrier, or it can be enclosed in hard or soft shell gelatin capsules, or it can be compressed into tablets, or it can be incorporated directly with the food of the diet. For oral administration, the infant dietary supplement composition may be incorporated with excipient and used in the form of ingestible tablets, buccal tablets, troches, capsules, elixirs, suspensions, syrups, wafers, and the like. The tablets, troches, pills, capsules and the like can also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin can be added or a flavoring agent such as peppermint, oil of wintergreen, or cherry flavoring. When the dosage unit form is a capsule, it can contain, in addition to materials of the above type, a liquid carrier. Various other materials can be present as coatings or to otherwise modify the physical form of the dosage unit. For instance, tablets, pills, or capsules can be coated with shellac, sugar or both. A syrup or elixir can contain the active compound, sucrose as a sweetening agent, methyl and propylparabens a preservatives, a dye and flavoring such as cherry or orange flavor. Oil in water emulsions may be better suited for oral use in infants because these are water-miscible and thus their oiliness is masked. Such emulsions are well known in the pharmaceutical sciences.

### EXAMPLES

### Example 1: DHA Bioequivalence Study in Adults

This Example shows dose dependent retroconversion of DPA(n-6) to ARA in the presence of DHA in adults.

To study the effects of DHA-rich *Schizochytrium* (Martek DHA™-S) oil and DHA-rich *Crypthecodinium* (DHASCO®) oil supplementation on plasma fatty acid content, healthy adults having an average age of approximately 38 years were randomized into treatment groups supplemented with gelatin capsules delivering either 0, 200, 600 or 1,000 mg DHA per day for a total of 4 weeks. This study was an eight-arm, prospective, randomized, parallel group, bioequivalence study using three doses of DHA (200, 600 or 1000 mg DHA per day) from either DHASCO® or Martek DHA™-S capsules, placebo capsules or a single dose of DHA (465 mg per day) from Martek DHA™-S nutritional bars for a four-week treatment period. Among groups consuming capsules, the study was double-blind and placebo controlled.

Blood samples were collected at baseline and following four weeks of DHA supplementation. Plasma was isolated, purged with N₂ and stored at -80°C until fatty acid analysis. Plasma lipids were extracted using the method of Folch and phospholipids were isolated by thin layer chromatography (TLC) on silica gel plates developed in a 60:40:3 ratio solution (v/v/v) of hexane:ether:acetic acid. Internal standard (23:0 fatty acid) was added and the phospholipids were saponified with 0.5N NaOH in methanol. The resulting fatty acids were methylated using 14% BF3 in methanol and then extracted with hexane. The fatty acid methyl esters were separated by capillary column gas chromatography on an Agilent Series 6890 System equipped with a 30m FAMEWAX™ (Restek, State College, PA) column using a 48:1 split flow ratio with helium as a carrier gas and a programmed temperature gradient (130 to 250°C). Fatty acid methyl esters were identified by flame ionization detection and comparison of retention times to a mixed fatty acid methyl ester standard from NuChek Prep (Elysian, MN). Fatty acids were quantified by comparison to the 23:0 internal standard. Individual fatty acid levels are reported as grams per 100 g total fatty acids.

DHA-rich *Crypthecodinium* oil (DHASCO® oil) contains approximately 400 mg/g DHA, 0 mg/g DPA(n-6), 0 mg/g EPA, and 2.5 mg/g cholesterol, with the balance of common saturated and unsaturated fats, and minor sterol and carotenoid components. DHA-rich *Schizochytrium* oil (Martek DHA™-S Oil) contains approximately 400 mg/g DHA, 150 mg/g DPA(n-6), 11 mg/g EPA, and 2.5 mg/g cholesterol, with the balance of common saturated and unsaturated fats, and minor sterol and carotenoid components. Subject weights ranged from roughly 40 to 116.4 kg, with an average of 76.7 kg. Weight-normalized average DHA intake levels were calculated to be 2.6 mg DHA/kg/day at the 200 mg DHA/day level, 7.8 mg DHA/kg/day at the 600 mg DHA/day level and 13.0 mg DHA/kg/day at the 1,000 mg DHA/day level.

Supplementation with both the DHASCO® and Martek DHA™-S capsules resulted in a statistically significant (p<0.05), dose-dependent increase in plasma PL DHA levels as shown in **Figure 1****.** DPA(n-6) increased with the Martek DHA™-S treatment, but decreased with the DHASCO® treatment as shown in **Figure 2****.**

The response of ARA plasma phospholipids to DHA supplementation differed for the two DHA treatments as shown in **Table 1** and **Figure 3****.**

**Table 1. Summary of Differences in Measured Plasma Phospholipids for Example 1 DHA Bioavailability Study using DHA-rich Schizochytrium Oil (Martek DHA™-S oil) and DHA-rich Crypthecodinium Oil (DHASCO®) (n=12 individuals per cell) (represented as average of (mean 4 week sample - mean 0 week sample)/mean 0 week sample)**

| **Fatty Acid** | **Change in Fatty Acid % 200 mg DHA/day** | **Change in Fatty Acid % 600 mg DHA/day** | **Change in Fatty Acid (%) 1,000 mg DHA/day** |
|---|---|---|---|
| *DHA-rich Schizochytrium Oil (Martek DHA*™-*S oil)* | | | |
| Arachidonic acid (C20:4 n-6) | 1% | -11% | -4% |
| Docosapentaenoic acid (C22:5 n-6) | 71% | 72% | 103% |
| Docosahexaenoic acid (C22:6 n-3) | 46% | 65% | 109% |
| *DHA-rich Crypthecodinium Oil (DHASCO® oil)* | | | |
| Arachidonic acid (C20:4 n-6) | -2% | -11% | -14% |
| Docosapentaenoic acid | -16% | -39% | -48% |
| (C22:5 n-6) | | | |
| Docosahexaenoic acid (C22:6 n-3) | 35% | 83% | 69% |

For the DHASCO® treatment, plasma phospholipid ARA levels decreased in a manner inversely proportional to DHA dose. For the Martek DHA™-S treatment, however, plasma phospholipid ARA levels showed a similar decrease of 11% at the 600 mg/day dose, however, ARA levels decreased by only 4% at the highest 1000 mg DHA/day dose. A decrease in ARA content with DHA supplementation is expected and commonly observed due to inhibition of ARA synthesis by DHA. The near maintenance of ARA levels observed at the higher 1000 mg DHA/day dose with the Martek DHA™-S Oil was not expected and can best be explained by *in vivo* retroconversion of DPA(n-6) to ARA at this dose.

### Example 2: DHA-S Bioavailability Study in Children

This example shows dose dependent retroconversion of DPA(n-6) to ARA in the presence of DHA. To study the effects of DHA-rich *Schizochytrium* oil (Martek DHA™-S Oil) supplementation on plasma fatty acid content, healthy children aged 4 to 6 years were randomized into two treatment groups supplemented with of chewable gelatin capsules delivering either 200 mg or 400 mg DHA per day for a total of 4 weeks. There were approximately 20 subjects per treatment group. The source of DHA was the DHA-rich triglyceride oil derived from *Schizochytrium* sp. (Martek DHA™-S Oil), which contains approximately 400 mg/g DHA, 150 mg/g DPA(n-6), 11 mg/g EPA, and 2.5 mg/g cholesterol, with the balance of common saturated and unsaturated fats, and minor sterol and carotenoid components. Subject weights ranged from 15.3 to 25.4 kg, with an average of 18.8 kg. Weight-normalized average DHA intake levels were calculated to be 10.6 mg DHA/kg/day at the 200 mg DHA/day level and 21.3 mg DHA/kg/day at the 400 mg DHA/day level. Blood samples were taken before treatment began and after the 4 week treatment period ended. Plasma phospholipid fatty acids were measured as described in Example 1 for each sample and tabulated as shown in **Figure 4****.** The change in key fatty acids over the 4 week time period were calculated by subtracting the plasma phospholipid fatty acid content at 4 weeks from the corresponding value at 0 weeks, and averaging these differences. These results are summarized in **Table 2.**

**Table 2. Summary of Differences in Measured Plasma Phospholipids for DHA Bioavailability Study in Children using DHA-rich Schizochytrium Oil (n = 10-12 per group) (represented as average of (4 week sample - 0 week sample)/0 week sample)**

| **Fatty Acid** | **Change in Fatty Acid % 200 mg DHA/day** | **Change in Fatty Acid (%) 400 mg DHA/day** | |
|---|---|---|---|
| Arachidonic acid (C20:4 n-6) | -13%* | -7%* | |
| Docosapentaenoic acid (C22:5 n-6) | +45%* | +71%* | |
| Docosahexanoic acid (C22:6 n-3) | +130%* | +242%* | |

| | | | |
|---|---|---|---|
| * indicates results which are significantly different (p-value < 0.05) | | | |

The results show behavior similar to that observed in **Example 1.** Both the DHA and DPA(n-6) phospholipid contents increased in a dose dependent manner, confirming subject compliance with the treatment protocol. ARA in plasma phospholipids decreased by an average of 13% at the 200 mg DHA/day dose, however, decreased by only 7% at the higher 400 mg DHA/day dose. A decrease in ARA content with DHA supplementation is expected and commonly observed due to inhibition of ARA synthesis by DHA. That lower levels of ARA decline were observed at the higher 400 mg DHA/day dose was not expected and can best be explained by *in vivo* retroconversion of DPA(n-6) to ARA. This result is consistent with the results observed and **Example 1**, especially when compared on a weight-normalized dose basis (mg DHA/kg/day).

The results from Examples 1 and 2 demonstrate how the use of the DHA-rich *Schizochytrium* oil (Martek DHA™-S Oil), which also contains DPA(n-6), can reduce the need for co-supplementation of ARA with DHA toward a goal of increasing serum DHA without decreasing serum ARA. These results show that a significant suppression of ARA decrease comes at a weight normalized dose of approximately 10-25 mg DHA/kg/day which corresponds to a DPA(n-6) dose of 2-3 to about 6-8 mg DPA(n-6)/kg/day.

### Example 3. Maintenance of ARA levels by DPA(n-6) in a rats in vivo

Sprague Dawley rats were fed an AIN-76A purified diet containing various amounts between 0 to 3% of Martek DHA™-S oil or DHASCO® oil for 28 days after which blood was collected. Plasma was harvested and stored at -80°C until fatty acid analysis. Fatty acid levels were determined in whole plasma. Briefly, internal standard (23:0) was added to the plasma samples and lipids were extracted using the method of Folch. Extracted lipids were saponified with 0.5N NaOH in methanol, and the resulting fatty acids were methylated using 14% BF3 in methanol and then extracted with hexane. The fatty acid methyl esters were separated by capillary column gas chromatography on an Agilent Series 6890 System equipped with a 30m FAMEWAX™ (Restek, State College, PA) column using a 48:1 split flow ratio with helium as a carrier gas and a programmed temperature gradient (130 to 250°C). Fatty acid methyl esters were identified by flame ionization detection and comparison of retention times to a mixed fatty acid methyl ester standard from NuChek Prep (Elysian, MN). Fatty acids were quantified by comparison to the 23:0 internal standard. Individual fatty acid levels are reported as grams per 100 g total fatty acids.

Figures 5A-C depict DHA (A), DPA(n-6) (B) and ARA (C) levels in plasma following 28 days of feeding rats with a diet containing Martek DHA™-S oil or DHASCO® oil. Graphs are normalized to DHA dose on a mg per kg body weight per day basis. Note that the DHA levels increased in a similar dose dependent manner with both the Martek DHA™-S oil and DHASCO® oil, but that the Martek DHA™-S oil resulted in an increase in DPA(n-6) levels (due to the presence of this fatty acid in the oil) whereas the DHASCO® oil led to a decrease in DPA(n-6) levels. The ARA levels decreased in a smooth DHA dose dependent manner with the DHASCO® oil. However, supplementation with the Martek DHA™-S oil, which contains DPA(n-6), resulted in a curvilinear ARA response, with reductions in ARA at low doses but a near maintenance of ARA levels at the higher doses, presumably due to the retroconversion of DPA(n-6) to ARA after reaching threshold DPA(n-6) levels in vivo. The rat ARA response to DPA(n-6) parallels the response seen with DPA(n-6) supplementation in humans.

Example 4. Blending thraustochytrid and dinoflagellate oils to provide varied DHA:DPA(n-6) ratios for use in infant formula. This experiment describes providing blended oils in which the DHA : DPA(n-6) ratio is varied.

The blend composition needed to achieve a DHA/DPA(n-6) ratio of 4:1 was determined by solving simultaneously three equations:

| | Martek DHA™-S | DHASCO® | Blend | |
|---|---|---|---|---|
| DHA balance: | 0.40% X + | 0.40%(1-X)= | D | (1) |
| DPA(n-6) balance | 0.15% X + | 0%(1-X) = | P | (2) |
| DHA/DPA(n-6) | D / P = 4 | | | (3) |

where:
   X = wt. fraction of Martek DHA™-S in the final blend
   Y = wt, fraction of DHASCO® in the final blend = 1 - X
   D = wt. fraction of DHA in the final blend
   P = wt. fraction of DPA in the final blend

Solving (1): D = 0.4
(3): P = 0.1
(2):
   X = 0.0.659 (Martek DHA™-S % in final blend)→ 65.9% Martek DHA™-S (*Schizochytrium* oil)
   Y = 0.0.341 (DHASCO® % in final blend) → 34.1% DHASCO® (*Crypthecodinium* oil)

Similarly, other target DHA/DPA(n-6) ratios can be calculated by the same method, taking into account the amount of DHA and DPA(n-6) in the starting oils. For example, for a blend of DHASCO® Oil, or Martek DHA™-S Oil, the following target DHA/DPA(n-6) ratios can be calculated:

| | | | |
|---|---|---|---|
| Target DHA/DPA(n-6) | 3.5 | 10.0 | 3.0 |
| % of DHASCO® Oil | 25% | 74% | 12% |
| % of Martek DHA™-S Oil | 75% | 26% | 88% |

The foregoing description of the present invention has been presented for purposes of illustration and description. Furthermore, the description is not intended to limit the invention to the form disclosed herein. Consequently, variations and modifications commensurate with the above teachings, and the skill or knowledge of the relevant art, are within the scope of the present invention. The embodiment described hereinabove is further intended to explain the best mode known for practicing the invention and to enable others skilled in the art to utilize the invention in such, or other, embodiments and with various modifications required by the particular applications or uses of the present invention. Further, elements from one embodiment can be readily recombined with elements from one or more other embodiments. Such combinations can form a number of embodiments within the scope of the invention. It is intended that the appended claims be construed to include alternative embodiments to the extent permitted by the prior art. Moreover, various other aspects of the invention disclosed herein are set out in the following numbered paragraphs (paras).
1. An infant formula composition, wherein, when ready for consumption by the infant, the composition comprises long chain n-3 fatty acids and long chain n-6 fatty acids; wherein the long chain n-3 fatty acids comprise docosahexaenoic acid (DHA); wherein the long chain n-6 fatty acids comprise docosapentaenoic acid (DPA(n-6)) and optionally arachidonic acid (ARA); wherein the ratio of ARA:DHA is less than about 3:1, and wherein the DHA:DPA(n-6) ratio is from about 5:1 to about 0.5:1.
2. The infant formula composition of para 1, wherein the ratio of ARA:DHA is less than about 1.95:1.
3. The infant formula composition of para 1, wherein the ratio of ARA:DHA is less than about 1.85:1.
4. The infant formula composition of para 1, wherein the ratio of ARA:DHA is less than about 1.75:1.
5. The infant formula composition of para 1, wherein the ratio of ARA:DHA is greater than about 0.4:1.
6. The infant formula composition of para 1, wherein the long chain n-6 fatty acids in the infant formula compositions are supplied in a source oil, wherein the source oil comprises long chain n-6 fatty acids comprising at least about 2% by weight DPA(n-6).
7. The infant formula composition of para 1, wherein the composition comprises at least about 7 mg/L of DPA(n-6).
8. The infant formula composition of para 1, wherein the DHA, ARA or DPA(n-6) is from a source selected from the group consisting of a plant, an oilseed, a microorganism, an animal, and mixtures of the foregoing.
9. The infant formula composition of para 8, wherein the source is a microorganism selected from the group consisting of algae, bacteria, fungi and protists.
10. The infant formula composition of para 8, wherein the source is selected from the group consisting of genetically modified plant and genetically modified oilseed selected from the group consisting of soybean, corn, safflower, sunflower, canola, flax, peanut, mustard, rapeseed, chickpea, cotton, lentil, white clover, olive, palm, borage, evening primrose, linseed and tobacco and mixtures thereof.
11. The infant formula composition of para 8, wherein the source is selected from the group consisting of a genetically modified plant, a genetically modified oilseed, and a genetically modified microorganism, wherein the genetic modification comprises the introduction of a polyketide synthase gene or a portion thereof.
12. The infant formula composition of para 8, wherein the source is a microorganism selected from the group consisting of Thraustochytriales, dinoflagellates, and *Mortierella.*
13. The infant formula composition of para 12, wherein the microorganism is selected from the group consisting of *Schizochytrium, Thraustochytrium,* and *Crypthecodinium.*
14. The infant formula composition of para 1, wherein the composition comprises a blend of microbial oils, and wherein the blend comprises oil from a thraustochytrid microorganism and oil from a dinoflagellate microorganism.
15. The infant formula composition of para 1, wherein the DHA:DPA(n-6) ratio is selected from the group consisting of from about 5:1 to about 3:1, from about 5:1 to about 2:1, and from about 5:1 to about 1:1.
16. The infant formula composition of para 1, wherein the composition further comprises from about 0.37 mg/L of cholesterol to about 500 mg/L cholesterol.
17. The infant formula composition of para 1, wherein the composition further comprises from about 0.37 mg/L of cholesterol to about 3.75 mg/L cholesterol.
18. The infant formula composition of para 1, wherein the composition comprises eicosapentaenoic acid (EPA) in an amount less than about 3 mg/L.
19. The infant formula composition of para 1, wherein the composition comprises EPA in an amount less than about 60 mg/L.
20. The infant formula composition of para 1, wherein the composition is formulated to provide at least about 2 mg/kg/day DPA(n-6) when administered to an infant.
21. The infant formula composition of para 20, wherein the composition is formulated to provide from about 3 mg/kg/day DPA(n-6) to about 8 mg/kg/day DPA(n-6) when administered to an infant.
22. A method of feeding an infant comprising administering an infant formula composition of any of paras 1-21 to an infant.
23. A method of preparing an infant formula composition, comprising combining nutritional components, long chain n-3 fatty acids and long chain n-6 fatty acids; wherein the long chain n-3 fatty acids comprise DHA; wherein the long chain n-6 fatty acids comprise ARA and DPA(n-6); wherein the ratio of ARA:DHA is less than about 3:1, and wherein the DHA:DPA(n-6) ratio is from about 5:1 to about 0.5:1.
24. An infant formula composition, wherein the composition comprises long chain n-6 fatty acids and DHA, wherein the long chain n-6 fatty acids are supplied in a source oil, wherein the source oil comprises long chain n-6 fatty acids comprising at least about 2% by weight DPA(n-6), wherein the DHA:DPA(n-6) ratio is from about 5:1 to about 0.5:1, and wherein the composition is formulated to provide at least about 2 mg/kg/day DPA(n-6) when administered to an infant.
25. The infant formula composition of para 24, wherein the long chain n-6 fatty acids in the source oil comprise at least about 5% by weight DPA(n-6).
26. The infant formula composition of para 24, wherein the long chain n-6 fatty acids in the source oil comprise at least about 10% by weight DPA(n-6).
27. The infant formula composition of para 24, wherein the long chain n-6 fatty acids in the source oil comprise at least about 15% by weight DPA(n-6).
28. The infant formula composition of para 24, wherein the long chain n-6 fatty acids in the source oil comprise at least about 20% by weight DPA(n-6).
29. The infant formula composition of para 24, wherein the long chain n-6 fatty acids in the source oil comprise at least about 25% by weight DPA(n-6).
30. The infant formula composition of para 24, wherein the long chain n-6 fatty acids comprise ARA; and wherein the ratio of ARA:DHA is less than about 3:1.
31. The infant formula composition of para 30, wherein the ratio of ARA:DHA is greater than about 0.4:1.
32. The infant formula composition of para 24, wherein the composition does not comprise ARA.
33. The infant formula composition of para 24, wherein the composition comprises at least about 7 mg/L of DPA(n-6).
34. The infant formula composition of para 24, wherein the DHA or DPA(n-6) is from a source selected from the group consisting of a plant, an oilseed, a microorganism, an animal, and mixtures of the foregoing.
35. The infant formula composition of para 34, wherein the source is a microorganism selected from the group consisting of algae, bacteria, fungi and protists.
36. The infant formula composition of para 34, wherein the source is selected from the group consisting of genetically modified plant and genetically modified oilseed selected from the group consisting of soybean, corn, safflower, sunflower, canola, flax, peanut, mustard, rapeseed, chickpea, cotton, lentil, white clover, olive, palm, borage, evening primrose, linseed and tobacco and mixtures thereof.
37. The infant formula composition of para 34, wherein the source is selected from the group consisting of a genetically modified plant, a genetically modified oilseed, and a genetically modified microorganism, wherein the genetic modification comprises the introduction of a polyketide synthase gene or a portion thereof.
38. The infant formula composition of para 34, wherein the source is a microorganism selected from the group consisting of Thraustochytriales, dinoflagellates, and *Mortierella.*
39. The infant formula composition of para 38, wherein the microorganism is selected from the group consisting of *Schizochytrium, Thraustochytrium,* and *Crypthecodinium.*
40. The infant formula composition of para 24, wherein the composition comprises a blend of microbial oils, and wherein the blend comprises oil from a thraustochytrid microorganism and oil from a dinoflagellate microorganism.
41. The infant formula composition of para 24, wherein the DHA:DPA(n-6) ratio is selected from the group consisting of from about 5:1 1 to about 3:1, from about 5:1 to about 2:1, and from about 5:1 to about 1:1.
42. The infant formula composition of para 24, wherein the composition further comprises from about 0.37 mg/L of cholesterol to about 500 mg/L cholesterol.
43. The infant formula composition of para 24, wherein the composition further comprises from about 0.37 mg/L of cholesterol to about 3.75 mg/L cholesterol.
44. The infant formula composition of para 24, wherein the composition comprises EPA in an amount less than about 3 mg/L.
45. The infant formula composition of para 24, wherein the composition comprises EPA in an amount less than about 60 mg/L.
46. The infant formula composition of para 24, wherein the composition is formulated to provide from about 3 mg/kg/day DPA(n-6) to about 8 mg/kg/day DPA(n-6) when administered to an infant.
47. A method of feeding an infant comprising administering an infant formula composition of any of paras 24-46 to an infant.
48. A method of preparing an infant formula composition, comprising combining nutritional components, DHA, and the long chain n-6 fatty acids, wherein the long chain n-6 fatty acids in the infant formula compositions are supplied in a source oil, wherein the source oil comprises long chain n-6 fatty acids comprising at least about 2% by weight DPA(n-6), and wherein the DHA:DPA(n-6) ratio is from about 5:1 to about 0.5:1, whereby an infant formula composition is prepared that provides at least about 2 mg/kg/day DPA(n-6) when administered to an infant.
49. An infant formula composition, wherein the composition is formulated to comprise at least about 7 mg/L of DPA(n-6) when ready for consumption, wherein the composition further comprises long chain n-3 fatty acids; wherein the long chain n-3 fatty acids comprise DHA; wherein the composition further optionally comprises arachidonic acid ARA; and wherein the ratio of ARA:DHA is less than about 3:1.
50. The infant formula composition of para 49, wherein the ratio of ARA:DHA. is greater than about 0.4:1.
51. The infant formula composition of para 49, wherein the composition does not comprise ARA.
52. The infant formula composition of para 49, wherein the composition comprises at least about 13 mg/L of DPA(n-6).
53. The infant formula composition of para 49, wherein the composition comprises at least about 26 mg/L of DPA(n-6).
54. The infant formula composition of para 49, wherein the composition comprises at least about 40 mg/L of DPA(n-6).
55. The infant formula composition of para 49, wherein the composition comprises at least about 53 mg/L of DPA(n-6).
56. The infant formula composition of para 49, wherein the composition comprises at least about 66 mg/L of DPA(n-6).
57. The infant formula composition of para 49, wherein the DPA(n-6) in the composition is supplied in a source oil, wherein the source oil comprises long chain n-6 fatty acids comprising at least about 2% by weight of long chain n-6 fatty acids.
58. The infant formula composition of para 49, wherein the DPA(n-6) is from a source selected from the group consisting of a plant, an oilseed, a microorganism, an animal, and mixtures of the foregoing.
59. The infant formula composition of para 58, wherein the source is a microorganism selected from the group consisting of algae, bacteria, fungi and protists.
60. The infant formula composition of para 58, wherein the source is selected from the group consisting of genetically modified plant and genetically modified oilseed selected from the group consisting of soybean, corn, safflower, sunflower, canola, flax, peanut, mustard, rapeseed, chickpea, cotton, lentil, white clover, olive, palm, borage, evening primrose, linseed and tobacco and mixtures thereof.
61. The infant formula composition of para 58, wherein the source is selected from the group consisting of a genetically modified plant, a genetically modified oilseed, and a genetically modified microorganism, wherein the genetic modification comprises the introduction of a polyketide synthase gene or a portion thereof.
62. The infant formula composition of para 49, wherein the source is a microorganism selected from the group consisting of Thraustochytriales, dinoflagellates, and *Mortierella.*
63. The infant formula composition of para 62, wherein the microorganism is wherein the microorganism is selected from the group consisting of *Schizochytrium, Thraustochytrium,* and *Crypthecodinium.*
64. The infant formula composition of para 49, wherein the composition comprises a blend of microbial oils, and wherein the blend comprises oil from a thraustochytrid microorganism and oil from a dinoflagellate microorganism.
65. The infant formula composition of para 49, wherein the DHA:DPA(n-6) ratio is selected from the group consisting of from about 5:1 to about 3:1, from about 5:1 to about 2:1, and from about 5:1 to about 1:1.
66. The infant formula composition of para 49, wherein the composition further comprises from about 0.37 mg/L of cholesterol to about 500 mg/L cholesterol.
67. The infant formula composition of para 49, wherein the composition further comprises at from about 0.37 mg/L of cholesterol to about 3.75 mg/L cholesterol.
68. The infant formula composition of para 49, wherein the composition comprises EPA in an-amount less than about 3 mg/L.
69. The infant formula composition of para 49, wherein the composition comprises EPA in an amount less than about 60 mg/L.
70. The infant formula composition of para 49, wherein the composition is formulated to provide at least about 2 mg/kg/day DPA(n-6) when administered to an infant.
71. The infant formula composition of para 70, wherein the composition is formulated to provide from about 3 mg/kg/day DPA(n-6) to about 8 mg/kg/day DPA(n-6) when administered to an infant.
72. A method of feeding an infant comprising administering an infant formula composition of any of paras 49-71 to an infant.
73. A method of preparing an infant formula composition, comprising combining nutritional components, DPA(n-6), DHA, and optionally, ARA; wherein the composition is formulated to comprise at least about 7 mg/L of DPA(n-6) when ready for consumption, and wherein the ratio of ARA:DHA is less than about 3:1.
74. An infant formula composition comprising a blend of microbial oils, wherein the blend comprises a first oil comprising DHA and DPA(n-6) and a second oil comprising DHA.
75. An infant formula composition comprising a blend of microbial oils, wherein the blend comprises an oil having a first ratio of DHA and DPA(n-6) and second oil having a second DHA ratio DPA(n-6), wherein the ratios are different and wherein the first oil has more DPA(n-6) than the second oil.
76. An infant formula composition comprising a blend of microbial oils, wherein the blend comprises a first oil from a thraustochytrid microorganism and a second oil from a dinoflagellate microorganism.
77. The infant formula composition of para 76, wherein the thraustochytrid microorganism is of the genus *Schizochytrium* and the dinoflagellate microorganism is of the genus *Crypthecodinium.*
78. The infant formula composition of para 76, wherein the blend comprises between about 10% by weight and about 90% by weight oil from a thraustochytrid microorganism and between about 90% by weight and about 10% by weight oil from a dinoflagellate microorganism.
79. The infant formula composition of para 76, wherein the blend comprises between about 50% by weight and about 82% by weight oil from a thraustochytrid microorganism and between about 18% by weight and about SO% by weight oil from a dinoflagellate microorganism.
80. The infant formula composition of para 76, wherein the blend comprises between about 57.5% by weight and about 67.5% by weight oil from a thraustochytrid microorganism and between about 32.5% by weight and about 42.5% by weight oil from a dinoflagellate microorganism.
81. The infant formula composition of any one of paras 75-80, wherein the blend has a DHA: DPA(n-6) ratio of between about 3:1 and about 10:1.
82. The infant formula composition of any one of paras 75-80, wherein the blend has a DHA: DPA(n-6) ratio of between about 0.5:1 and about 10:1.
83. The infant formula composition of any one of paras 75-80, wherein the blend has a DHA: DPA(n-6) ratio of between about 3.5:1 and about 4.5:1.
84. The infant formula composition of any one of paras 75-80, wherein the blend comprises long chain n-6 fatty acids and wherein the long chain n-6 fatty acids are supplied in a source oil, wherein the source oil comprises long chain n-6 fatty acids comprising at least about 2% by weight DPA(n-6).
85. The infant formula composition of any one of paras 75-80, wherein the composition comprises long chain n-3 fatty acids and long chain n-6 fatty acids; wherein the long chain n-3 fatty acids comprise DHA; wherein the long chain n-6 fatty acids comprise arachidonic acid ARA; and wherein the ratio of ARA:DHA is less than about 3:1.
86. The infant formula composition of para 85, wherein the ratio of ARA:DHA is greater than about 0.4:1.
87. The infant formula composition of para 85, wherein the composition does not comprise ARA.
88. The infant formula composition of any one of paras 75-80, wherein the composition comprises at least about 7 mg/L of DPA(n-6).
89. The infant formula composition of any one of paras 75-80, wherein the DHA:DPA(n-6) ratio is selected from the group consisting of from about 5:1 to about 3:1, from about 5:1 to about 2:1, and from about 5:1 1 to about 1:1.
90. The infant formula composition of any one of paras 75-80, wherein the composition further comprises from about 0.37 mg/L of cholesterol to about 500 mg/L cholesterol.
91. The infant formula composition of any one of paras 75-80, wherein the composition further comprises at from about 0.37 mg/L of cholesterol to about 3.75 mg/L cholesterol.
92. The infant formula composition of any one of paras 75-80, wherein the composition comprises EPA in an amount less than about 3 mg/L.
93. The infant formula composition of any one of paras 75-80, wherein the composition comprises EPA in an amount less than about 60 mg/L.
94. The infant formula composition of any one of paras 75-80, wherein the composition is formulated to provide at least about 2 mg/kg/day DPA(n-6) when administered to an infant.
95. The infant formula composition of para 93, wherein the composition is formulated to provide from about 3 mg/kg/day DPA(n-6) to about 8 mg/kg/day DPA(n-6) when administered to an infant.
96. A method of feeding an infant comprising administering an infant formula composition of any of paras 75-95 to an infant.
97. A method of preparing an infant formula composition, comprising combining nutritional components, and a blend of microbial oils, wherein the blend is selected from the group consisting of
   a blend comprising a first oil comprising DHA and DPA(n-6) and a second oil comprising DHA;
   a blend comprising an oil having a first ratio of DHA and DPA(n-6) and second oil having a second DHA ratio DPA(n-6), wherein the ratios are different and wherein the first oil has more DPA(n-6) than the second oil; and
   a blend comprising a first oil from a thraustochytrid microorganism and a second oil from a dinoflagellate microorganism.
98. An infant formula composition comprising a blend of microbial and plant oils, wherein the blend comprises oil from a plant genetically modified to produce DHA and DPA(n-6), and wherein the plant oil comprises DHA and DPA(n-6), and wherein the microbial oil comprises ARA.
99. The infant formula of para 98, wherein the genetic modification comprises the introduction of a PUFA polyketide synthase gene or portions thereof.
100. An infant formula composition comprising a plant oil from a plant genetically modified to produce DHA and DPA(n-6), and wherein the oil comprises DHA and DPA(n-6) in a ratio of from about 0.5:1 to about 5:1.
101. The infant formula of para 100, wherein the genetic modification comprises the introduction of a PUFA polyketide synthase gene or portions thereof.
102. An infant formula composition comprising a microbial oil from a genetically modified microorganism and wherein the oil comprises DHA and DPA(n-6) in a ratio of from about 0.5:1 to about 5:1.
103. The infant formula of para 102, wherein the genetic modification comprises the introduction of a PUFA polyketide synthase gene or portions thereof.
104. An infant formula composition formulated to meet DHA and ARA target requirements and to maintain normal growth, weight, nervous system development or immune system development, wherein the composition comprises;
   DHA in the target DHA requirement amount;
   ARA in a sub-target amount less than the target ARA requirement amount; and
   DPA(n-6) in an amount sufficient to retroconvert to ARA *in vivo,* in an amount sufficient to offset the ARA sub-target amount.
105. An infant formula composition formulated to meet DHA and ARA target requirements and to maintain normal growth, weight, nervous system development or immune system development, wherein the composition comprises;
   DHA in the target DHA requirement amount;
   ARA in a sub-target amount less than the target ARA requirement amount; and DPA(n-6) in at least a threshold amount, wherein the ARA response curve is curvilinear, and wherein the threshold amount results in an ARA response at which the slope of the ARA response curve changes from negative to positive.
106. The infant formula composition of para 104 or 105, wherein the ARA sub-target amount is an amount selected from the group consisting of an amount at least about 5% below the target ARA requirement amount, an amount at least about 10% below the target ARA requirement amount, an amount at least about 15% below the target ARA requirement amount, and an amount about 20% below the target ARA requirement amount.
107. The infant formula composition of para 104 or 105, wherein the DPA(n-6) is supplied in a source oil, wherein the source oil comprises long chain n-6 fatty acids comprising at least about 2% by weight of total long chain n-6 fatty acids.
108. The infant formula composition of para 104 or 105, wherein the DPA(n-6) comprise at least about 5% by weight of total long chain n-6 fatty acids in the composition.
109. The infant formula composition of para 104 or 105, wherein the DPA(n-6) comprise at least about 10% by weight of total long chain n-6 fatty acids in the composition.
110. The infant formula composition of para 104 or 105, wherein the DPA(n-6) comprise at least about 15% by weight of total long chain n-6 fatty acids in the composition.
111. The infant formula composition of para 104 or 105, wherein the DPA(n-6) comprise at least about 20% by weight of total long chain n-6 fatty acids in the composition.
112. The infant formula composition of para 104 or 105, wherein the DPA(n-6) comprise at least about 25% by weight of total long chain n-6 fatty acids in the composition.
113. The infant formula composition of para 104 or 105, wherein the ratio of ARA:DHA is less than about 3:1.
114. The infant formula composition of para 104 or 105, wherein the ratio of ARA:DHA is less than about 1.95:1.
115. The infant formula composition of para 104 or 105, wherein the ratio of ARA:DHA is less than about 1.85:1.
116. The infant formula composition of para 104 or 105, wherein the ratio of ARA:DHA is less than about 1.75:1.
117. The infant formula composition of para 104 or 105, wherein the ratio of ARA:DHA is greater than about 0.4:1.
118. The infant formula composition of para 104 or 105, wherein the composition does not comprise ARA.
119. The infant formula composition of para 104 or 105, wherein the composition comprises at least about 7 mg/L of DPA(n-6).
120. The infant formula composition of para 104 or 105, wherein the composition comprises at least about 66 mg/L of DPA(n-6).
121. The infant formula composition of para 104 or 105, wherein the composition comprises from about 40 to about 140 mg/L of DHA.
122. The infant formula composition of para 104 or 105, wherein the DHA or DPA(n-6) is from a source selected from the group consisting of a plant, an oilseed, a microorganism, an animal, and mixtures of the foregoing.
123. The infant formula composition of para 122, wherein the source is a microorganism selected from the group consisting of algae, bacteria, fungi and protists.
124. The infant formula composition of para 122, wherein the source is selected from the group consisting of genetically modified plant and genetically modified oilseed selected from the group consisting of soybean, corn, safflower, sunflower, canola, flax, peanut, mustard, rapeseed, chickpea, cotton, lentil, white clover, olive, palm, borage, evening primrose, linseed and tobacco and mixtures thereof.
125. The infant formula composition of para 122, wherein the source is selected from the group consisting of a genetically modified plant, a genetically modified oilseed, and a genetically modified microorganism, wherein the genetic modification comprises the introduction of a polyketide synthase gene or a portion thereof.
126. The infant formula composition of para 122, wherein the source is a microorganism selected from the group consisting of Thraustochytriales, dinoflagellates, and *Mortierella.*
127. The infant formula composition of para 126, wherein the microorganism is selected from the group consisting of *Schizochytrium, Thraustochytrium,* and *Crypthecodinium.*
128. The infant formula composition of para 104 or 105, wherein the composition comprises a blend of microbial oils, and wherein the blend comprises oil from a thraustochytrid microorganism and oil from a dinoflagellate microorganism.
129. The infant formula composition of para 104 or 105, wherein the DHA:DPA(n-6) ratio is from about 10:1 to about 0.5:1.
130. The infant formula composition of para 104 or 105, wherein the DHA:DPA(n-6) ratio is from about 5:1 to about 0.5:1.
131. The infant formula composition of para 104 or 105, wherein the DHA:DPA(n-6) ratio is selected from the group consisting of from about 5:1 to about 3:1, from about 5:1 to about 2:1, and from about 5:1 to about 1:1.
132. The infant formula composition of para 104 or 105, wherein the composition further comprises from about 0.37 mg/L of cholesterol to about 500 mg/L cholesterol.
133. The infant formula composition of para 104 or 105, wherein the composition further comprises at from about 0.37 mg/L of cholesterol to about 3.75 mg/L cholesterol.
134. The infant formula composition of para 104 or 105, wherein the composition comprises EPA in an amount less than about 3 mg/L.
135. The infant formula composition of para 104 or 105, wherein the composition comprises EPA in an amount less than about 60 mg/L.
136. The infant formula composition of para 104 or 105, wherein the composition is formulated to provide at least about 2 mg/kg/day DPA(n-6) when administered to an infant.
137. The infant formula composition of para 104 or 105, wherein the composition is formulated to provide from about 3 mg/kg/day DPA(n-6) to about 8 mg/kg/day DPA(n-6) when administered to an infant.
138. A method of feeding an infant comprising administering an infant formula composition of any of paras 104-137 to an infant, whereby normal growth, weight, nervous system development or immune system development is maintained.
139. A method of preparing an infant formula composition, comprising combining nutritional components, DHA, ARA, and DPA(n-6), wherein DHA is present in a target DHA requirement amount; ARA is present in a sub-target amount less than a target ARA requirement amount; and DPA(n-6) is present in an amount selected from the group consisting of
   an amount sufficient to retroconvert to ARA *in vivo*, in an amount sufficient to offset the ARA sub-target amount: and
   at least a threshold amount, wherein the ARA response curve is curvilinear, and wherein the threshold amount results in an ARA response at which the slope of the ARA response curve changes from negative to positive.
140. The method of para 139, wherein the ARA sub-target amount is an amount selected from the group consisting of an amount about 5% below the target ARA requirement amount, an amount about 10% below the target ARA requirement amount, an amount about 15% below the target ARA requirement amount, and an amount about 20% below the target ARA requirement amount.
141. A method of feeding an infant comprising administering an infant formula composition containing nutritional components, DHA and DPA(n-6) to an infant, wherein the infant is fed at least about 2 mg DPA(n-6)/kg/day.
142. The method of para 141, wherein the infant formula composition is the infant formula composition of any of paras 1-21, 24-45, 49-71, or 104-137.
143. A premature infant nutrition fortifier composition formulated for combination with an infant milk or formula, wherein the premature infant nutrition fortifier composition comprises DPA(n-6) and wherein when combined with the infant or formula, the combination comprises DPA(n-6) in a threshold amount.
144. The premature infant nutrition fortifier composition of para 143, further comprising DHA.
145. The premature infant nutrition fortifier composition of para 144, further comprising ARA.
146. The premature infant nutrition fortifier composition of para 144, wherein the ratio of DHA:DPA(n-6) ratio is from about 5:1 to about 0.5:1.
147. The premature infant nutrition fortifier composition of para 143, further comprising a component selected from the group consisting of protein, fat, vitamins and minerals.
148. A method of providing supplemental nutrients to a preterm infant comprising administering an infant milk or formula fortified with the premature infant nutrition fortifier composition of claim composition of any of paras 144-147 to a preterm infant.
149. A method of promoting growth of a premature infant comprising administering an infant milk or formula fortified with the premature infant nutrition fortifier composition of claim composition of any of paras 144-147 to a preterm infant
150. A method of preparing a fortified premature infant milk or formula, comprising combining an infant milk or formula with the composition of any of paras 144-147.
151. An infant dietary supplement composition, wherein the infant dietary supplement composition comprises DPA(n-6) and an excipient, wherein the combination comprises DPA(n-6) in a threshold amount.
152. The infant dietary supplement composition of para 151, further comprising DHA.
153. The infant dietary supplement composition of para 152, further comprising ARA.
154. The infant dietary supplement composition of para 152, wherein the ratio of DHA:DPA(n-6) ratio is from about 5:1 to about 0.5:1.
155. The infant dietary supplement composition of para 151, further comprising a component selected from the group consisting of protein, fat, vitamins and minerals.
156. A method of providing supplemental nutrients to an infant comprising administering an infant dietary supplement composition of any of paras 151-155 to an infant.

## Claims

1. A method of feeding an infant, comprising administering an infant formula composition containing nutritional components, docosahexaenoic acid (DHA), and docosapentaenoic acid (DPA(n-6)) to an infant, wherein the infant is fed at least about 2 mg DPA(n-6)/kg/day.

2. The method of claim 1, wherein the composition is formulated to provide from about 3 mg/kg/day DPA(n-6) to about 8 mg/kg/day DPA(n-6) when administered to an infant.

3. A premature infant nutrition fortifier composition formulated for combination with an infant milk or formula, wherein the premature infant nutrition fortifier composition comprises DPA(n-6), wherein when combined with the infant or formula, the combination comprises DPA(n-6) in a threshold amount.

4. The premature infant nutrition fortifier composition of claim 3, further comprising DHA.

5. The premature infant nutrition fortifier composition of claim 3, further comprising arachidonic acid (ARA).

6. The premature infant nutrition fortifier composition of claim 4, wherein the ratio of DHA:DPA(n-6) ratio is from about 10:1 to about 0.5:1.

7. The premature infant nutrition fortifier composition of claim 3, further comprising a component selected from protein, fat, vitamins, and minerals.

8. The use of a premature infant nutrition fortifier composition of any one of claims 3-7 in the preparation of a product for providing supplemental nutrients to a preterm infant.

9. The use of a premature infant nutrition fortifier composition of any one of claims 3-7 in the preparation of a product for promoting growth of a preterm infant.

10. A method of preparing a premature infant milk or formula, comprising combining an infant milk or formula and the premature infant nutrition fortifier composition of any one of claims 3-7.

11. An infant dietary supplement composition, wherein the infant dietary supplement composition comprises DPA(n-6) and an excipient, wherein the combination comprises DPA(n-6) in a threshold amount.

12. The infant dietary supplement composition of claim 11, further comprising DHA.

13. The infant dietary supplement composition of claim 11, further comprising ARA.

14. The infant dietary supplement composition of claim 12, wherein the ratio of DHA:DPA(n-6) ratio is from about 10:1 to about 0.5:1.

15. The infant dietary supplement composition of claim 11, further comprising a component selected from protein, fat, vitamins, and minerals.

16. The use of an infant dietary supplement composition of any one of claims 11-15 in the preparation of a product for providing supplemental nutrients to an infant.
